(19) Europäisches Patentamt
European Patent Office
Office européen des brevets

(11) **EP 2 793 850 B1**

(12) **EUROPÄISCHE PATENTSCHRIFT**

(45) Veröffentlichungstag und Bekanntmachung des Hinweises auf die Patenterteilung:
**16.11.2016 Patentblatt 2016/46**

(21) Anmeldenummer: **12700468.7**

(22) Anmeldetag: **09.01.2012**

(51) Int Cl.:
*A61K 9/00* (2006.01)       *A61B 5/06* (2006.01)
*G01R 33/09* (2006.01)       *A61B 1/04* (2006.01)

(86) Internationale Anmeldenummer:
**PCT/EP2012/050217**

(87) Internationale Veröffentlichungsnummer:
**WO 2013/091901 (27.06.2013 Gazette 2013/26)**

(54) **DETEKTIONSSYSTEM ZUR ERFASSUNG MAGNETISCHER OBJEKTE IM MENSCHLICHEN ORGANISMUS**

DETECTION SYSTEM FOR DETECTING MAGNETIC OBJECTS IN THE HUMAN ORGANISM

SYSTÈME DESTINÉ À DÉTECTER DES OBJETS MAGNÉTIQUES DANS L'ORGANISME HUMAIN

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **21.12.2011 DE 102011089334**

(43) Veröffentlichungstag der Anmeldung:
**29.10.2014 Patentblatt 2014/44**

(73) Patentinhaber: **Evonik Röhm GmbH 64293 Darmstadt (DE)**

(72) Erfinder:
• **HARTWIG, Benedikt 64287 Darmstadt (DE)**
• **NIEPOTH, Peter 64823 Groß-Umstadt (DE)**

• **JUNGINGER, Steffen 18059 Rostock (DE)**
• **STILLER, Hans-Joachim verstorben (DE)**
• **WINDHAB, Norbert 65719 Hofheim (DE)**
• **GEIPEL, Gerhard 45721 Haltern am See (DE)**

(56) Entgegenhaltungen:
**WO-A1-97/09640**

• **CHAO HU ET AL: "A Cubic 3-Axis Magnetic Sensor Array for Wirelessly Tracking Magnet Position and Orientation", IEEE SENSORS JOURNAL, IEEE SERVICE CENTER, NEW YORK, NY, US, Bd. 9, Nr. 5, 1. Mai 2010 (2010-05-01), Seiten 903-913, XP011306917, ISSN: 1530-437X**

EP 2 793 850 B1

**Beschreibung**

Die Erfindung betrifft die technische Ausführung eines Sensorarrangements, das magnetische oder magnetisierte orale Darreichungsformen nach der oralen Einnahme detektieren kann und deren Auflösung über die Abnahme oder das Verschwinden des magnetischen Feldes der oralen Darreichungsform verfolgt.

Techniken zur Messung magnetischer Flüsse sind seit Langem bekannt. Es gibt Sensoren, die auf relativ kleinem Raum mit hoher Empfindlichkeit den Vektor der magnetischen Flussdichte messen können, beispielsweise Sensoren auf dem Funktionsprinzip des anisotropen magnetoresistiven Effektes, abgekürzt AMR-Effekt. Beim AMR-Effekt ist in bestimmten Materialien der ohmsche Widerstand vom Winkel zwischen dem Stromfluss und dem Magnetisierungsvektor abhängig. Besonders gut zu beobachten ist er in dünnen Schichten aus Permalloy, einer Legierung aus 81% Ni und 19% Fe. In kommerziellen Sensoren werden vier Einzelwiderstände zu einer Wheatstone-Brücke verschaltet. Mit der sogenannten Barberpole-Struktur wird ein Winkel von 45° zwischen dem Magnetisierungsvektor und dem durch den jeweiligen Widerstand fließenden Strom erzwungen. Der Magnetisierungsvektor ist bei fehlendem äußerem Magnetfeld in Richtung der Längsachse des Widerstandes, der sogenannten Achse der leichten Magnetisierung, ausgerichtet. Wird ein äußeres Magnetfeld angelegt, so wird der Magnetisierungsvektor um einen Winkel gegen diese Achse gedreht. Dadurch ändert sich der Winkel zwischen Stromfluss und Magnetisierungsvektor, was wiederum mit einer Änderung des ohmschen Widerstands verbunden ist.

Die Patentanmeldung WO 2011/026808 A1 offenbart ein Computer-basiertes Auswertungssystem, das den magnetischen Fluss einer oralen Darreichungsform erfasst, die mit magnetisierten Phasen ausgestattet ist. Die verschiedenen Phasen der Darreichungsform können so ausgeführt sein, dass diese sich nach der oralen Einnahme zu unterschiedlichen Zeiten im menschlichen Verdauungssystem auflösen. Das Auswertungssystem misst das aus der Überlagerung der magnetischen Phasen resultierende Magnetfeld diskontinuierlich mithilfe eines Hall Sensors, der auf einer Petrischale fixiert ist. Unter der Voraussetzung, dass Magnetfelder im menschlichen Körper in einem Abstand von 5 - 20 cm bis zur Hautoberfläche erfasst werden, erzeugt das System anhand des resultierenden Magnetfeldes eine Signatur, mit deren Erfassung man den Zeitpunkt der Inkorporation und der Auflösung, aber auch die charakteristische Ausführung der Darreichungsform identifiziert. Doch werden keine Angaben darüber gemacht, wie ein solches Signal gewonnen werden kann, wenn sich der Mensch mitsamt des Auswertungssystems bewegt, und auch nicht darüber, wie ein verwertbares Signal erkannt werden kann, wenn störende Magnetfelder mitgemessen werden, die nicht von der Darreichungsform ausgehen.

In dem Aufsatz mit dem *Titel "*Every slow-wave impulse is associated with motor activity of the human stomach" vom 8. Dezember 2008 bei der American Physiological Society beschreiben Michael Hocke, Ulrike Schöne et al. ein System, das die Bewegungen kleiner magnetischer Marker im menschlichen Magen erfasst. Während der Patient ruhig liegen muss, wird das stationäre System, das aus 9 Magnetfeldsensoren besteht, in Position gebracht. Sobald weder störende Magnetfelder vorhanden sind, noch sogenannte "Artefakte", die nicht eindeutig dem Marker zugeordnet werden können, können die Messungen durchgeführt werden.

Ein anderes Detektorsystem zur Erfassung magnetischer Körper im menschlichen Organismus wird in dem Aufsatz "A Cubic 3-Axis Magnetic Sensor Array for Wirelessly Tracking Magnet Position and Orientation" von Chao Hu und Kollegen in IEEE Sensors Journal Vol. 10, No. 5, May 2010 beschrieben. Das Detektorsystem beinhaltet mindestens 2 AMR Sensoranordnungen.

Aufgabe der vorliegenden Erfindung war es also, mit Hilfe eines Systems kleine Änderungen in dem Magnetfeld zu erfassen, das im Alltagsgeschehen durch einen verabreichten magnetischen Körper hervorgerufen wird. Eine weitere Aufgabe bestand in der Bereitstellung eines Verfahrens zur Gewinnung eines Meßsignals und dessen Auswertung, mit welchem ein verabreichter magnetischer Körper im menschlichen Organismus im Alltagsgeschehen nachgewiesen wird. Im Rahmen der Erfindung wird das störende Magnetfeld der Umgebung zwar homogen, doch nicht zeitlich konstant angenommen. Denn die Sensoranordnung und der magnetische Körper können sich im umgebenden Magnetfeld bewegen. Zusätzlich bewegt sich der magnetische Körper relativ zur Sensoranordnung (Schlucken, Lageänderungen im Magen) und verändert seine magnetischen Eigenschaften durch Desintegration mit einer vorgegebenen Zeitabhängigkeit. Des Weiteren treten zahlreiche Objekte auf, die das Magnetfeld der Umgebung verändern, beispielsweise Fahrzeuge, metallische Möbel, stromdurchflossene Leitungen und dgl. mehr. Die magnetische Flussdichte des Umgebungsfeldes, etwa des Erdmagnetfeldes, liegt typischerweise bei 35 µT. Im Rahmen der Erfindung wird von der Messung magnetischer Flussdichten mit Hilfe eines Teslameters bei mindestens 1 cm Abstand ausgegangen.

Magnetische Körper, zum Beispiel zu Zylindern gepresstes Magnetit in den Abmessungen einiger mm, erzeugen auf den im Rahmen der Erfindung interessierenden Längenskalen von wenigen cm bis 0,5 m magnetische Flüsse von wenigen 100 nT. Die Kompensation störender Flüsse oder deren Erkennen im Meßsignal ist also für die Brauchbarkeit des Detektorsystems entscheidend.

Überraschend gelingt die Lösung der Aufgabe durch ein Detektorsystem aus mindestens zwei Sensoranordnungen, wobei jede Sensoranordnung einen, zwei, oder drei anisotrope Magnetwiderstandssensoren aufweist. Die Achsen der leichten Magnetisierung der Magnetwiderstandssensoren weisen in paarweise unterschiedliche Richtungen, und jede Sensoranordnung weist von der oder den übrigen Sensoranordnungen einen Abstand von 0,5 bis 50 cm auf. Zumindest zwei Sensoranordnungen sind in einem Winkel von 0 bis 45 ° gegeneinander verkippt.

Gegenstand der Erfindung ist also ein Detektorsystem zur Erfassung magnetischer Körper im menschlichen Organismus, das zumindest zwei Sensoranordnungen aufweist, wobei jede Sensoranordnung einen, zwei, oder drei anisotrope Magnetwiderstandssensoren aufweist, deren

Achsen der leichten Magnetisierung in paarweise unterschiedliche Richtungen weisen, und jede Sensoranordnung von der oder den übrigen Sensoranordnungen einen Abstand von 0,5 bis 50 cm aufweist, und zumindest zwei Sensoranordnungen in einem Winkel von 0 bis 45° gegeneinander verkippt sind.

Der Begriff "anisotroper Magnetwiderstandssensor" wird im Rahmen der Erfindung mit "AMR-Sensor" abgekürzt.

Die Erfindung wird im Folgenden näher erläutert.

Das erfindungsgemäße Detektorsystem wird mittels Gurt am Körper mitgeführt , denn der Gurt, in welchem die Sensoranordnungen integriert sein können, ist für das Tragen am Menschen vorgesehen, und wird ohne oder mit Hilfe eines Dritten am Menschen angelegt .

Dieser Gurt kann beispielsweise ein Gürtel sein, der seinen Träger nur minimal in seinen alltäglichen Bewegungen einschränkt. Der Gurt kann vorteilhaft ein kombinierter Brust- und Schultergurt sein. Besonders vorteilhaft kann der kombinierte Brust- und Schultergurt ein Gurtsystem sein, das aus dem Klettersport bekannt ist. Der kombinierte Brust- und Schultergurt hat den Vorteil, den Menschen beim Tragen nicht in seinen alltäglichen Bewegungen einzuschränken und die Sensoranordnungen mit hoher Genauigkeit relativ zur Speiseröhre und dem Magen-Darm-Trakt zu positionieren. Das Gurtsystem hat zusätzlich den besonderen Vorteil, die Sensoranordnungen des erfindungsgemäßen Detektorsystems besonders genau jeweils in definiertem Abstand und deren Achsen der leichten Magnetisierung in definiertem Winkel zu halten. Der Gurt erlaubt seinem Träger die volle Beweglichkeit bei den alltäglichen Verrichtungen, insbesondere bei Betätigungen in Beruf und Freizeit.

Weist eine Sensoranordnung des erfindungsgemäßen Detektorsystems lediglich einen AMR-Sensor auf, wird diese im Rahmen der Erfindung *auch "einkanalig" genannt,* bei drei AMR-Sensoren entsprechend *"dreikanalig".* Weist eine Sensoranordnung beispielsweise drei AMR-Sensoren auf, deren leicht magnetisierbare Achsen wie die Koordinatenachsen x, y, und z eines kartesischen Koordinatensystems angeordnet sind, so sind die Komponenten des Vektors dieser Sensoranordnung die Meßsignale in x, y, bzw. in z-Richtung die Signale $S_x$, $S_y$, und $S_z$. Sie sind das Maß für die magnetische Flussdichte in Richtung der Koordinatenachsen.

Die Achsen der leichten Magnetisierung einer Sensoranordnung treffen sich in einem gedachten Punkt, dem Ursprung der jeweiligen Sensoranordnung. Der Abstand zwischen diesen Ursprüngen, bzw. bei drei Sensoranordnungen der paarweise Abstand zwischen diesen Ursprüngen, ist im Rahmen der Erfindung der Abstand bzw. der paarweise Abstand zwischen den Sensoranordnungen.

Die Achsen der leichten Magnetisierung der zweiten Sensoranordnung liegen jeweils parallel zu den Koordinatenachsen x, y, und z, oder in einem Winkel dazu. Im Rahmen der Erfindung ist dieser Winkel wie folgt definiert: Die Achsen der leichten Magnetisierung jeder Sensoranordnung liegen auf je einem gedachten Kegelmantel eines Raumwinkels. Im Rahmen der Erfindung ist der Winkel, in dem die zwei Sensoranordnungen des erfindungsgemäßen Detektorsystems gegeneinander verkippt sind, der Winkel zwischen den Mittenachsen der Kegel der Sensoranordnungen.

Wird das Detektorsystem in einem Gurt mitgeführt, liegt der Winkel im Rahmen der Genauigkeit, mit der der Gurt eingestellt werden kann, in der Ebene, die durch die Ursprünge der Sensoranordnungen und den Eintrittspunkt der Speiseröhre in den Magen definiert ist. Besonders hohe Genauigkeiten werden erreicht, wenn dieser Gegenstand ein aus dem Klettersport bekanntes Gurtsystem ist.

Weist das erfindungsgemäße Detektorsystem zwei Sensoranordnungen auf, werden die Richtungen und die Signale durchnummeriert. Demnach erhält man in den Richtungen x1, y1, z1 bzw. x2, y2, z2 die Signale $S_{x1}$, $S_{y1}$, und $S_{z1}$ bzw. $S_{x2}$, $S_{y2}$, und $S_{z2}$, aus denen man die Vektoren $\boldsymbol{S_1}$ und $\boldsymbol{S_2}$ bildet:

$$\boldsymbol{S_1} = (S_{x1},\ S_{y1},\ S_{z1})\ ,$$

und

$$\boldsymbol{S_2} = (S_{x2},\ S_{y2},\ S_{z2})$$

Weist beispielsweise die erste der Sensoranordnungen des erfindungsgemäßen Detektorsystems nur einen AMR-Sensor auf, nämlich in Richtung x1, vereinfacht sich der Vektor $\boldsymbol{S_1}$ zu

$$\boldsymbol{S_1} = (S_{x1}, 0, 0)$$

Das erfindungsgemäße Detektorsystem hat den Vorteil, diese Vektorkomponenten jeweils so genau zu messen und derart auswertbar zu machen, dass bei der Bewegung der Sensoranordnung durch den Träger die Schwankung des Betrages dieser Vektoren entweder klein bleibt, oder soweit bekannt ist, dass die durch einen magnetischen Körper verursachte Veränderung der Messwerte erfasst wird. Somit wird der Einfluss äußerer Störquellen erkannt und beseitigt, oder kann aus dem Meßsignal gefiltert werden.

Die Beträge der Vektoren, abgekürzt mit $|\boldsymbol{S_1}|$ und $|\boldsymbol{S_2}|$, errechnet man auf bekannte Weise:

$$|\boldsymbol{S_1}| = (S_{x1}{}^2 + S_{y1}{}^2 + S_{z1}{}^2)^{1/2} \, ,$$

$$|\boldsymbol{S_2}| = (S_{x2}{}^2 + S_{y2}{}^2 + S_{z2}{}^2)^{1/2}$$

Bei geringem Abstand zwischen den Sensoranordnungen ergeben sich in homogenen Feldern gleiche Messwerte. Ein magnetischer Körper mit geringer magnetischer Induktion in der Nähe der Sensoren beeinflusst durch sein mit dem Abstand zum Sensor rasch abklingendes Magnetfeld bei unterschiedlichen Abständen zu den Sensoren deren Messwerte unterschiedlich. Da aber jede Sensoranordnung einen Vektor liefert, der sich aus den Meßsignalen der AMR-Sensoren zusammensetzt, hat das erfindungsgemäße Detektorsystem den Vorteil, dass sich die Nähe des magnetischen Körpers zu den Sensoranordnungen auf den Winkel zwischen den gemessenen Vektoren auswirkt. Bewegt sich der magnetische Körper, ändert sich dieser Winkel.

Die Messempfindlichkeit kann durch vorteilhafte Ausführungen des erfindungsgemäßen Detektorsystems erhöht werden.

Vorzugsweise weist zumindest einer, bevorzugt jeder AMR-Sensor des Detektorsystems 4 Barberpole-Elemente auf, die zu einer Wheatstonebrücke oder einer Wheatstonebrückenersatzschaltung zusammengeschaltet sind. Die Achse der leichten Magnetisierung ist dann die Resultierende der Achsen der leichten Magnetisierung der einzelnen Barberpole-Elemente. Äußere Magnetfelder verstimmen eine solche Wheatstonebrücke viel stärker, als z.B. eine Widerstandsbrücke mit nur einem Barberpole-Element und drei konventionellen ohmschen Widerständen. Die Empfindlichkeit einer Wheatstone-Brücke aus 4 Barberpole-Elementen ist demnach erhöht.

In der Fachwelt ist bekannt, dass die Kennlinie des AMR-Sensors durch starke Magnetfelder verändert werden kann, weil sich Domänen des anisotropen Materials umbilden oder verformen, oder weil sich deren Wände in dem Material verlagern. Diesem Effekt kann durch zumindest einen Set- oder/und Reset-Impuls entgegen gewirkt werden, der einmalig vor der Messung, vorzugsweise mehrmals während der Messung, besonders bevorzugt periodisch während der Messung über einen Set-Reset-Strap aufgegeben wird. Die Wirkung periodisch aufgegebener Set- und/oder Reset-Impulse besteht in der Sicherstellung der optimalen Kennlinie der AMR-Sensoren.

Ein Alternieren des Set- und Reset-Impulses, im Rahmen der Erfindung *"Flippen"* genannt, ermöglicht eine Beseitigung von Offsetfehlern durch Differenzbildung der nach jedem Impuls gemessenen Signale. Des Weiteren werden thermische, elektrische, und/oder solche Einflüsse eliminiert, die zum Beispiel bei der Erwärmung des AMR-Sensors auftreten.

Ebenfalls wird mit dem *Flippen* eine automatische Einstellung des Arbeitspunktes des nachfolgenden Verstärkers ermöglicht, die im Rahmen der *Erfindung "switching feedback"* genannt wird. Neben dem Tastverhältnis ist auch das sichere Erreichen der Sättigungsinduktion durch die Set- und Reset-Impulse wichtig.

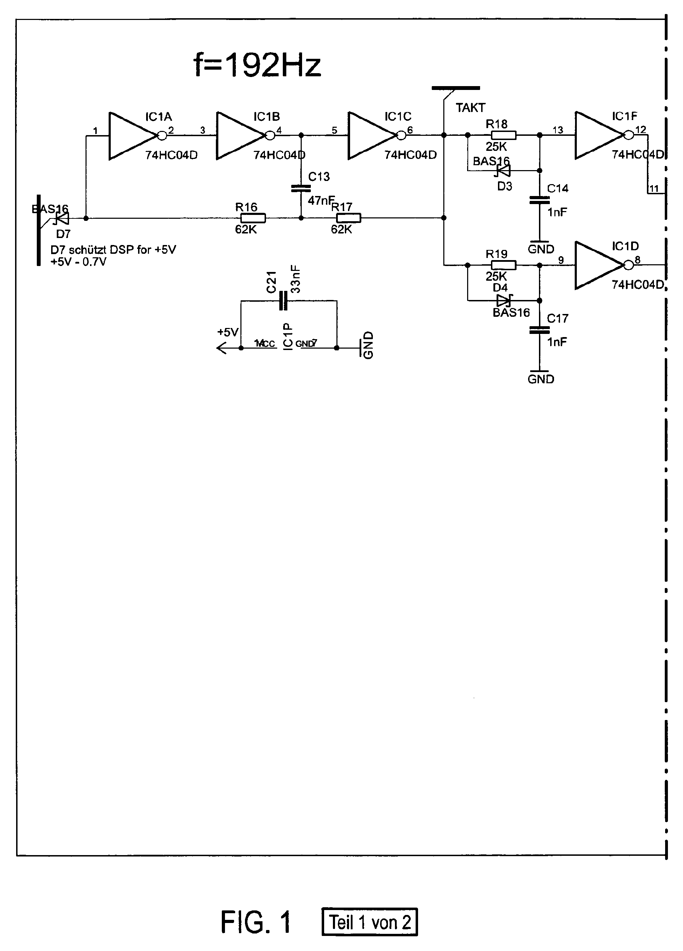

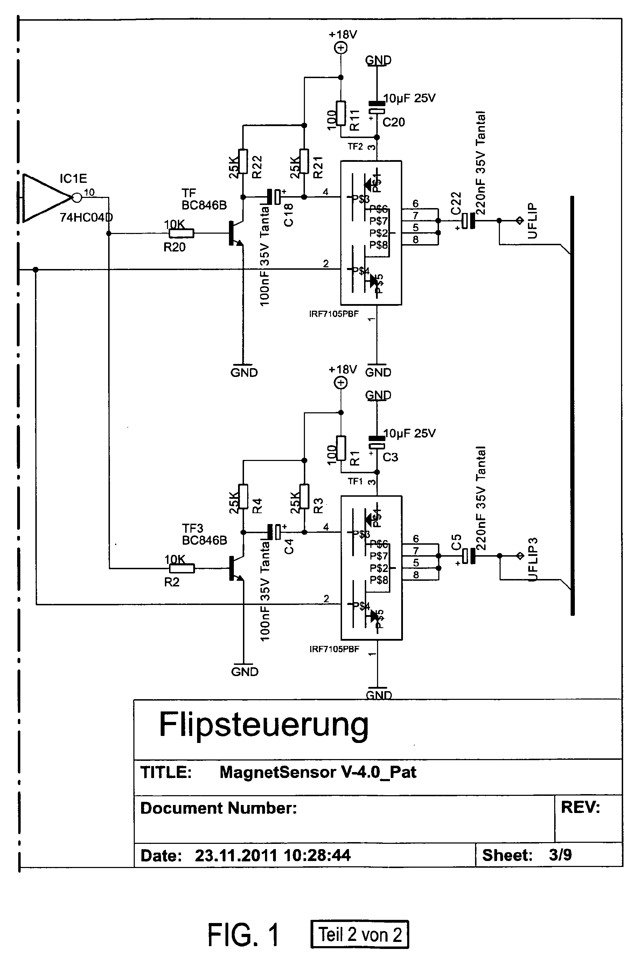

Die *Fig. 1* zeigt den Schaltplan, gemäß dem das *Flippen* ermöglicht wird. Bei der Differenzbildung muss der Arbeitspunkt für den nachfolgenden Verstärker eingestellt werden. Ungenauigkeiten dieser Einstellung wirken sich bei sehr großer Aussteuerung durch eine unsymmetrisch einsetzende Begrenzung des Signals aus.

Außerdem kann das erfindungsgemäße Detektorsystem einen Offset-Strap aufweisen. Der Strom durch den Offset-Strap kann durch eine Treiberschaltung geliefert werden, die z. B. einen Verstärker in Brückenschaltung als wesentliches Element enthalten kann. Der Offset-Strap ermöglicht die Kompensation der zu messenden Feldkomponente durch Erzeugung eines Feldes mit entgegengesetzter Ausrichtung. Ohne einen Offset-Strap muss bei der Messung der magnetischen Flussdichte die Nichtlinearität der Sensorkennlinie und außerdem die Querempfindlichkeit der AMR-Sensoren berücksichtigt werden. Die Querempfindlichkeit besteht in der Wirkung hoher Werte der magnetischen Flussdichte in sowohl einer Achsenrichtung, als auch auf den Messwert eines orthogonal dazu ausgerichteten AMR-Sensors.

Mit Offset-Strap jedoch wird die Brückenspannung des Sensors in einer Regelschleife durch Einspeisung eines Stroms in den Offset-Strap minimiert. Der für den Brückenabgleich erforderliche Strom im Offset-Strap ist ein Maß für das zu messende Feld. Dadurch wird stets in demjenigen Arbeitspunkt der Sensorkennlinie gemessen, in dem die Empfindlichkeit und Linearität ihr Maximum haben und gleichzeitig die Querempfindlichkeit verschwindet. Das erfindungsgemäße Detektorsystem ist deshalb für jede alltägliche Umgebung geeignet.

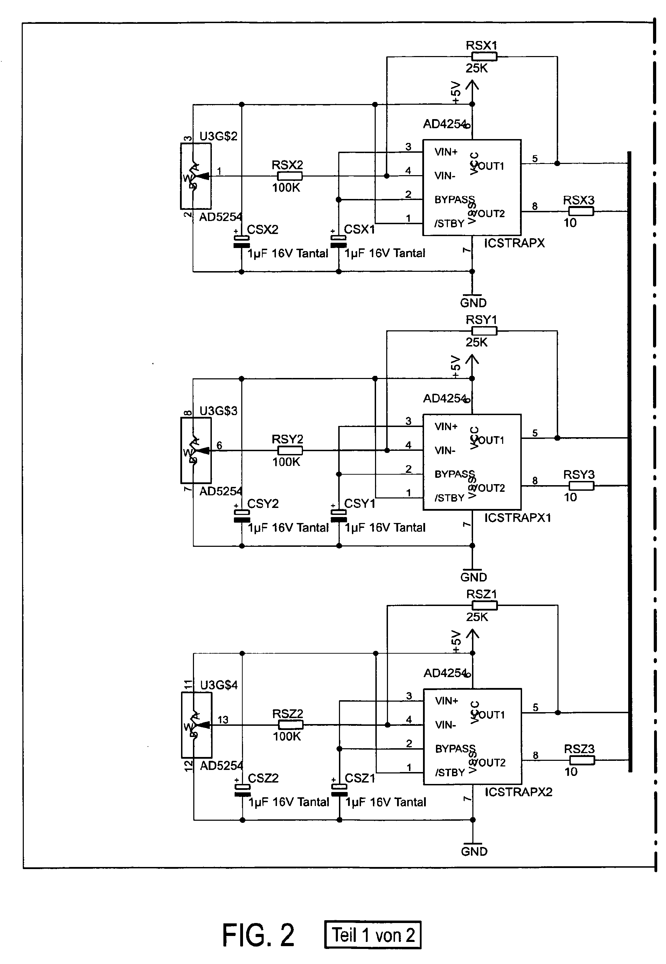

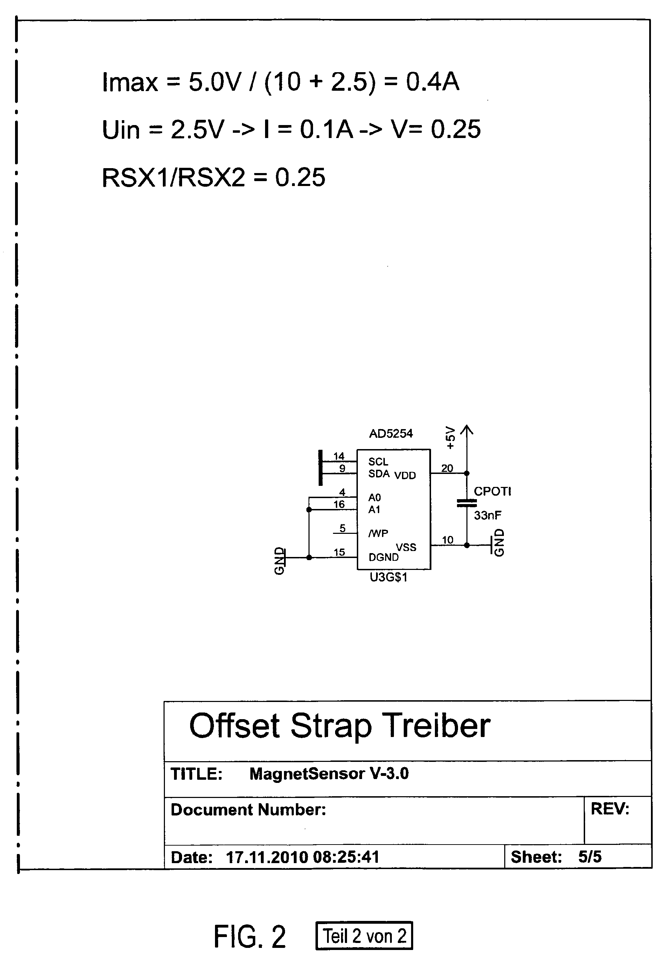

Der Offset-Strap ist mit dem "Offset Strap Treiber" beschaltet. Den Schaltplan zeigt die *Fig. 2.*

Allgemein können Nichtlinearitäten und Querempfindlichkeiten bei der Kalibrierung erfasst und das Messergebnis entsprechend korrigiert werden. Dadurch ist auch ein Betrieb ohne Ansteuerung des Offset-Straps bei minimiertem Energieverbrauch möglich.

Es gibt eine weitere Alternative zu der Kompensation der zu messenden Feldkomponente durch Erzeugung eines Feldes mit entgegengesetzter Ausrichtung mittels der Einspeisung eines Stroms in den Offset-Strap. Dabei kann zumindest ein, vorzugsweise jeder AMR-Sensor des erfindungsgemäßen Detektorsystems mit einer alternativen Schaltung ausgestattet sein.

Bei dieser Ausführungsform des Detektorsystems wird die Brückenspannung des Sensors nicht in einer Gegenkopplungsschaltung auf den Sollwert Null ausgeregelt. Stattdessen wird mittels eines DA-Wandlers und eines Verstärkers ein definierter Strom in den Offset-Strap in der Art eingespeist, dass ein bestimmter Aussteuerbereich der Sensorbrücke nicht verlassen wird.

Bei einer weiteren Möglichkeit der Ausführung des erfindungsgemäßen Detektorsystems kann der Aussteuerbereich der Sensorkennlinie in eine Anzahl von Segmenten unterteilt werden, beispielsweise bei einem DA-Wandler mit 8 Bit Auflösung in 256 Segmente. Zur Gewährleistung einer kontinuierlichen Messung bei sich ändernder magnetischer Feldstärke können die Segmente so gewählt werden, dass eine ausreichende Überlappung benachbarter Segmente vorliegt. Jedes dieser Segmente kann dann mit nur einem kleinen Aussteuerbereich um den optimalen Arbeitspunkt des AMR-Sensors versehen werden. Die Verringerung des Aussteuerbereiches verringert die Querempfindlichkeit und die Auswirkungen von Nichtlinearitäten der Kennlinie. Auf die vollständige Korrektur von Nichtlinearität und Querempfindlichkeit wird verzichtet. Dafür jedoch wird eine verbesserte Amplitudenauflösung der Messung durch die Kombination aus AD-Wandler und Segmentierung der Kennlinie erhalten.

Dafür müssen für jedes der Segmente eines AMR-Sensor Messbereiches die Parameter der Approximation durch jeweils eine Gerade mit ihrer dazu gehörenden Steigung und Höhenabschnitt bestimmt werden. Die Steigungen und Höhenabschnitte der Segmente werden über die Kalibrierungsdateien der Sensoren bereitgestellt. Wird das erfindungsgemäße Detektorsystem nun im täglichen Gebrauch bewegt, etwa durch die alltäglichen Bewegungen seines Trägers, so wird der definierte Strom und damit die Approximation ständig nachgeführt.

Entsprechend der Geschwindigkeit, mit der die Bewegungen erfolgen, ist eine hohe Abtastrate vorteilhaft, so dass ohne Übersteuerungen eine kontinuierliche Messung realisiert ist.

Der Vorteil dieser Variante besteht darin, dass bei entsprechend schneller Abtastung die Offsetstraps nur mit einem sehr kleinen Tastverhältnis betrieben werden müssen. Dadurch werden der Leistungsbedarf und die Eigenerwärmung der Sensoren und damit verbundene Offsetprobleme stark verringert.

Außerdem kann durch die Verwendung schneller AD- und DA-Wandler bei der für die kontinuierliche Messung im Magnetfeld erforderlichen Messfrequenz die für die einzelne Messung benötigte Zeit gering gehalten werden. Damit ist es möglich, die Offsetstraps nur während der für die Messwerterfassung erforderlichen Zeit anzusteuern. Erfolgt die Ansteuerung der Offsetstraps zum Beispiel nur mit einem Tastverhältnis von 0,1, zum Beispiel bei 1 ms Messdauer und einem zeitlichen Abstand zwischen aufeinander folgenden Messungen von 10 ms, so verringert sich die Verlustleistung. Dadurch wird weniger Wärme entwickelt und somit die Drift der Meßsignale vermindert oder ganz unterdrückt.

Für die Brauchbarkeit des erfindungsgemäßen Detektorsystems mit zwei Sensoranordnungen ist zu beachten, dass die Speiseröhre eine Länge von 20 bis 30 cm hat und von einem geschluckten Objekt in 5 bis 10 s durchlaufen wird. Daraus ergibt sich ein Geschwindigkeitsbereich bei der Speiseröhrenpassage von 2 bis 6 cm/s und damit ein entsprechend schnell veränderliches Signal für das Detektorsystem. Der Frequenzbereich des Nutzsignals deckt sich also mit dem Frequenzbereich, den einige der externen Störsignale besitzen. Im Rahmen der Erfindung werden "*externe Störsignale"* solche Signale genannt, die von magnetischen Flüssen herrühren, die den Träger umgeben und in denen er sich - zwangsweise - bewegt, zum Beispiel im Erdmagnetfeld oder in der Umgebung magnetischer Objekte wie z. B. Fahrzeuge. Aufgrund externer Störsignale würde man eine Unterscheidbarkeit zwischen einer Passage eines magnetischen Objektes durch die Speiseröhre und magnetischen Flüssen anderer Objekte nicht erwarten. Insbesondere führt eine Stand der Technik gemäße Filterung des Meßsignals nicht zum Erfolg.

Eine bekannte Möglichkeit zur Ausschaltung externer Störungen bietet sich durch die Auswertung von Auto- und Kreuzkorrelationsfunktionen von Sensoren, die in einem festen Abstand zu einander positioniert sind. Die Kreuzkorrelation beschreibt die Korrelation zweier Signale in Abhängigkeit von der Zeitverschiebung zwischen diesen Signalen. Bei der Autokorrelation wird die Korrelation eines Signals mit sich selbst berechnet. Die Autokorrelationsfunktion hat stets ein Maximum bei der Verschiebung 0. Wird ein Signal mit einer Verzögerung durch zwei sonst gleiche Sensoren aufgenommen, so ist das Maximum der Kreuzkorrelationsfunktion bei sonst gleicher Form um die Verzögerung gegenüber dem Maximum der Autokorrelationsfunktionen verschoben.

Eine wesentliche Voraussetzung für die Identifizierung einer Kapselpassage durch die Speiseröhre ist es, dass die Sensoranordnungen eine zeitversetzte Komponente der Signale erfassen können. Das Problem, welches jedoch verbleibt, ist von der Bewegung der Sensoranordnung im umgebenden Erdmagnetfeld verursacht, die die Brauchbarkeit des erfindungsgemäßen Detektorsystems ja gerade ausmacht.

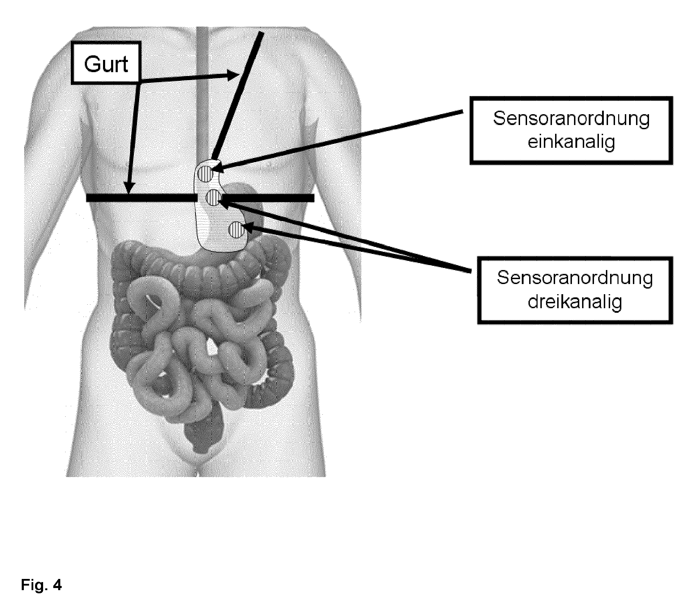

Es wurde überraschend gefunden, dass trotz einer Vielfalt magnetischer Flüsse zahlreicher Objekte, beispielsweise von Fahrzeugen, metallischen Möbeln, stromführenden Leitungen und Ähnlichem mehr, durch das Detektorsystem eindeutig solche Flüsse erkannt werden, die von dem magnetischen Körper im menschlichen Organismus stammen, wenn der Abstand zwischen zwei Sensoranordnungen von 2 bis 6 cm gewählt ist. Durch ungleiche Orte der Sensoranordnungen werden externe Magnetfelder erkannt, die nicht von dem magnetischen Körper im menschlichen Organismus stammen. Bevorzugt sind die Sensoranordnungen über Speiseröhre oder Brustbein und Magen senkrecht oder waagerecht fixiert. Die *Fig. 4.* zeigt das erfindungsgemäße Detektorsystem mit drei Sensoranordnungen in einem kombinierten Brust- und Schultergurt, getragen am Menschen. In dieser beispielhaften Ausführungsform ist die Sensoranordnung in Nähe der Speiseröhre einkanalig, die beiden anderen Sensoranordnungen sind dagegen dreikanalig ausgeführt. Die lediglich einkanalige Ausführung der Sensoranordnung in Nähe der Speiseröhre vereinfacht den Aufbau und mindert den Energiebedarf des erfindungsgemäßen Detektorsystems. Außerdem nutzt diese einkanalige Ausführungsform die Möglichkeit, dass der magnetische Körper nicht kugelsymmetrisch ausgeführt sein muss, sondern zum Beispiel zylindersymmetrisch ausgeführt sein kann, und das vom ihm erzeugte Magnetfeld sich dadurch während des Durchlaufens der Speiseröhre relativ zur einkanaligen Sensoranordnung bewegt, ohne zu rotieren.

Es kann auch vorteilhaft sein, den Anteil der störenden Umgebungsfelder durch die Subtraktion eines gleitenden Mittelwerts zu entfernen und den Abstand zwischen den Sensoranordnungen mit 2 cm zu wählen. Mit den gefilterten Signalen können dann deren Auto- und Kreuzkorrelationsfunktionen berechnet werden. Anhand der Unterschiede der Amplituden und der Lage der Maxima kann dann die Passage eines magnetischen Körpers erkannt werden.

Falls das erfindungsgemäße Detektorsystem zwei oder drei Sensoranordnungen aufweist, kann es für eine Erfassung des magnetischen Körpers im Magen verwendet werden.

Die langsame Desintegration des magnetischen Körpers führt zur Abschwächung von dessen magnetischer Flussdichte. Bewegungen des Trägers und Lageveränderungen des magnetischen Körpers, zum Beispiel durch die Peristaltik, führen zu Schwankungen des Messwertes. Obwohl im Allgemeinen keine Aussagen über das superponierte Bewegungsmuster aus Peristaltik und magnetischem Körper möglich sind, führt das Detektorsystem mit drei Sensoranordnungen zum Erfolg. Vorteilhaft ist des Weiteren, dieses Detektorsystem mit einer Tiefpassfilterung als Maßnahme für die Signalverarbeitung auszustatten.

Der magnetische Körper des erfindungsgemäßen Detektorsystems kann so ausgeprägt sein, dass er über die orale Einnahme verabreicht, insbesondere durch den Menschen geschluckt werden kann. Die Gestalt dieses magnetischen Körpers wird im Rahmen der Erfindung auch *"orale Darreichungsform"* genannt. Diese kann eine Kapsel oder eine Kapsel mit Funktion sein, wobei die Funktion ausgewählt ist aus Diagnostikum und/oder Arzneiform. Die Kapsel kann weiterhin bevorzugt eine Tablette sein, die die Speiseröhre vorzugsweise in Längsrichtung passiert. Die Darreichungsform weist zumindest einen magnetischen, vorzugsweise einen para-, superpara-, ferri-, und/oder ferromagnetischen Anteil auf, vorzugsweise zumindest einen Magnetit enthaltenden Kern und/oder Mantel. Der magnetische Anteil kann magnetisch orientierbare oder magnetisierbare Partikel aufweisen, vorzugsweise Magnetit ($Fe_3O_4$) oder Maghemit ($Fe_2O_3$). Magnetit und Maghemit gelten als toxikologisch und pharmakologisch unbedenklich und werden unter anderem als nicht toxische, unlösliche Pigmente in Lebensmittel oder Arzneiformen eingesetzt.

Geeignet können gegebenenfalls auch andere magnetisch orientierbare Partikel wie die Ferrite $MnFe_2O_4$ oder $MgFe_2O_4$ sein. Der magnetische Anteil des magnetischen Körpers kann in dem Bereich von 0,05 bis 80 mg, bevorzugt von 2 bis 70, bevorzugt 4 bis 60, insbesondere 6 bis 50 mg an magnetisch orientierbaren oder magnetisierbaren Partikeln betragen. Die mittleren Teilchengrößen der magnetisch orientierbare Partikel können z. B. im Bereich von 1 nm bis 1 mm, bevorzugt von 100 nm bis 100 $\mu$m liegen.

Die orale Darreichungsform kann ebenfalls bevorzugt eine Kapsel sein, eine Tablette, ein Stäbchen, eine überzogene Tablette, ein Schmelzextrudat, oder ein Körper mit einem eingearbeiteten magnetischen Film sein.

Damit erfasst das erfindungsgemäße Detektorsystem, bei welchem eine Sensoranordnung orthogonal zur Hauptachse der Darreichungsform ausgerichtet ist, eine markante Änderung des Messwertes bei ihrer Passage.

Die zeitliche und die räumliche Skala, auf der die Meßsignale zumindest der beiden Sensoranordnungen liegen, sind durch die Geschwindigkeit gegeben, mit der die orale Darreichungsform das erfindungsgemäße Detektorsystem passiert, und durch die Abstände bzw. paarweisen Abstände der Sensoranordnungen. Obwohl sich, wie oben bereits gesagt, eine Vielzahl magnetischer Flussdichten überlagern und die eigentlich interessierende Flussdichte sehr klein und zeitlich und räumlich inhomogen ist, wurde erkannt, dass diese sich mit dem erfindungsgemäßen Detektorsystem sicher erfassen lässt.

Deshalb ist ebenfalls Gegenstand der Erfindung ein Verfahren zur Erfassung der von einem magnetischen Körper im menschlichen Organismus erzeugten magnetischen Flussdichte durch das erfindungsgemäße Detektorsystem, wobei der magnetische Körper in oraler Darreichungsform vorliegt und ein Lebens- oder Genussmittel ist und einen magnetischen Anteil aufweist, welches durch die Schritte gekennzeichnet ist, dass

(a) zumindest einmal ein Set- und Reset-Impuls auf jeden anisotropen Magnetwiderstandssensor aufgeschaltet wird, und

(b) die Signale jedes AMR-Sensors über eine geeignete Signalaufbereitung und über zumindest einen Tiefpassfilter verstärkt werden, und anschließend

(c) die Differenz der Beträge der Vektoren der magnetischen Flussdichten jeder Sensoranordnung bestimmt wird, und/oder der Winkel $\Phi$ zwischen den Vektoren aus den Meßsignalen der AMR-Sensoren

errechnet wird.

Das Verfahren hat den Vorteil, bei der Erfassung von Messwerten dynamische Störeinflüsse zu mindern. Wird nämlich das Magnetfeld bei der Bestimmung des Offsetwertes durch äußere Einflüsse verzerrt, z. B. durch vorbeifahrende Fahrzeuge, so erhält der Fachmann verfälschte Offsetwerte. Eine weitere Gefahr der Verfälschung kennt der Fachmann durch das Ein- und Ausschwingverhalten von eingesetzten Filtern. Diese Effekte finden wir aber durch das erfindungsgemäße Verfahren verringert.

Im Schritt (a) sind die Set- und Reset-Impulse alternierend, gleichbedeutend damit, dass diese zyklisch aufgepulst werden. Sie sollten mit einer Stromimpulsstärke aufgegeben werden, bei der die Sättigungsmagnetisierung jeweils erreicht und damit die Steigung der Kennlinie kontrolliert wird. Die Stromimpulsstärke schwankt je nach Bauelement auf eine dem Fachmann bekannte Weise.

Im Schritt (b) können bevorzugt Gaußfilter, Besselfilter zum Unterdrücken von Überschwingern oder Welligkeiten im Signal eingesetzt werden. Um rasche und langsame Änderungen in den Signalen zu trennen, sind dem Fachmann bekannte Bandpassfilter eine bevorzugte Art der Signalaufbereitung. Periodische elektromagnetische Störungen mit Frequenzen von 16,7 Hz, z.B. beim elektrifizierten Bahnbetrieb, bzw. 50 Hz, der Netzfrequenz, können durch die Wahl der Abtastrate und der Integrationszeit von 60 ms oder vielfachen bei der Datenerfassung unterdrückt werden. Die Integrationszeit ist bei abweichenden Frequenzen der periodischen Störungen entsprechend anzupassen.

Um elektromagnetische Störeinstrahlungen der Frequenzbereiche von 16 bis 50 Hz auszufiltern, sind 2 Anordnungen bevorzugt, bei denen die Integrationskonstante mindestens 60 msec beträgt. Vorzugsweise werden so die Abtastfrequenzen an verschiedene, periodisch auftretende Störquellen angepasst.

Das Maß der magnetischen Flussdichte in x-, y-, und z-Richtung beim Schritt (c) sind die aus der Verstimmung der Wheatstone Brücken der AMR-Sensoren in der jeweiligen Richtung abfallenden Spannungen. Der Fachmann nimmt an, dass sich in der Differenz $\Delta_0$ der Vektoren zweier Sensoranordnungen,

$$\Delta_0 = S_1 - S_2,$$

die Anteile homogener magnetischer Flussdichten gerade aufheben. Der Einfluss störender externer Felder, räumlich kaum veränderlich, wäre damit kompensiert, und es bliebe im Wesentlichen das Feld des magnetischen Körpers im Träger allein übrig. Die beiden Sensoranordnungen dürfen dabei jedoch nicht oder nur wenig gegeneinander verkippt sein, gleichbedeutend mit dem Winkel 0°. Magnetische Flussdichten raum- und zeitversetzter Ereignisse werden jedoch überraschend auch bei größeren Winkeln erfasst, wenn anstelle $\Delta_0$ der skalare Wert $\Delta$ gebildet wird:

$$\Delta = |S_1| - |S_2|$$

Dies vereinfacht die Montage der Sensoranordnungen in dem Gurt des erfindungsgemäßen Detektorsystems und erspart außerdem umständliche Anpassungen der Lage der Sensoranordnungen an unterschiedliche Proportionen des Trägers. In einem Diagramm des Wertes $\Delta$ als Funktion der Zeit werden so charakteristische Linienformen erkannt, die zum Beispiel dem Schlucken des magnetischen Körpers, seinem Gang durch die Speiseröhre, somit die Passage der Sensoranordnungen, und seiner Bewegungen aufgrund der Peristaltik bei der Verdauung zugeordnet werden.

Um diese Zuordnung vornehmen zu können, ist die Filterung des Meßsignals nicht ausreichend. Zwar kennt der Stand der Technik eine Möglichkeit zur Ausschaltung externer Störungen durch die Auswertung von Auto- und Kreuzkorrelationsfunktionen von Sensoren, die in einem festen Abstand zueinander positioniert sind. Wird ein Signal mit einer Verzögerung durch zwei sonst gleiche Sensoren aufgenommen, so ist das Maximum der Kreuzkorrelationsfunktion bei sonst gleicher Form um die Verzögerung gegenüber dem Maximum der Autokorrelationsfunktionen verschoben. Damit nunmehr ein Zeitversatz zwischen Auto- und Kreuzkorrelation der Sensorsignale erkannt werden kann, darf der von der oralen Darreichungsform verursachte Signalanteil nicht durch externe Magnetfelder überdeckt werden. Dazu aber würde man die externen Störungen weitgehend beseitigen müssen. Hierzu wird beispielsweise die Differenzbildung zwischen aktuellem Signal und einem Mittelwert genutzt. Diesen Mittelwert muss man der aktuellen Situation anpassen und zum Beispiel als sogenannten "gleitenden Mittelwert (Moving Average)" gewinnen. Dies jedoch bedingt, dass der Proband während der Einnahme der oralen Darreichungsform weder schnelle rotatorische, noch rasche translatorische Bewegungen mit großer Amplitude vollzieht. Erst dann gewährleisten Sensoren gemäß Stand der Technik eine ausreichende Signaltrennung.

Natürlich kann man auch die Abtastrate erhöhen, so dass ohne Übersteuerungen eine kontinuierliche Messung realisiert wäre. Der Nachteil eines dann erhöhten Energiebedarfs kann wenigstens teilweise dadurch kompensiert werden, indem hohe Abtastraten nur bei interessanten, komplexen Ereignissen eingestellt werden, wie zum Beispiel beim Schlucken und/oder dem Zerfall des magnetischen Körpers. Solche interessanten, komplexen Ereignisse müssen von dem System allerdings erkannt werden. Das erfindungsgemäße Detektorsystem hat eine vorteilhafte Ausführungsform, bei der interessante, komplexe Ereignisse erkannt werden, nämlich anhand der Registrierung des genauen Zeitpunktes der Einnahme. Diese Ausführungsform wird weiter unten erläutert. Bei Stand der Technik gemäßen Lösungen besteht aber weiterhin das Problem, dass schnelle rotatorische und/oder translatorische Bewegungen mit großer Amplitude, die nicht mit der oralen Darreichungsform zusammenhängen, in den Meßsignalen weiterhin sichtbar sind.

Das alternative Berechnen des von den Meßsignalvektoren eingeschlossenen Winkels Φ nach der Formel I,

$$\text{I} \qquad \Phi = \arccos(S1 \cdot S2 \,/\, |S_1||S_2|),$$

im Schritt (c) des Verfahrens umgeht dieses Problem überraschend. Wir fanden, dass sich schnelle Bewegungen des Trägers und/oder rasche externe Flussänderungen der störenden Felder auf die relative Orientierung der Meßsignalvektoren zueinander weniger signifikant auswirken, als die Bewegung des magnetischen Körpers im Träger Organismus. Dies kann damit erklärt werden, dass die Quellen für externe Flussänderungen beide Meßsignalvektoren, oder bei drei Sensoranordnungen drei Meßsignalvektoren, in zumindest ungefähr gleiche Richtungen ablenken. Obwohl deren Beträge durchaus unterschiedlich verändert werden können, muss der Winkel zwischen je zwei Paaren der Meßsignalvektoren, auf die Zeit bezogen, in etwa der gleiche bleiben. Das ist gleichbedeutend damit, dass das umgebende Magnetfeld weiter entfernter Quellen seine Homogenität oder Inhomogenität in etwa beibehält. I erlaubt demnach das Ausblenden weiter entfernter Quellen magnetischer Flüsse unabhängig von deren zeitlichem Verhalten.

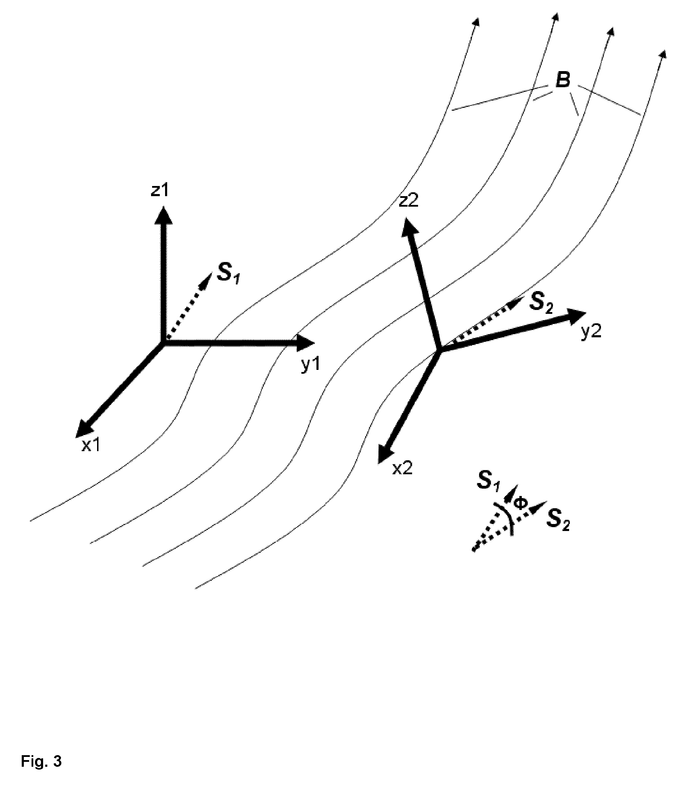

Die Situation bei der Durchführung des erfindungsgemäßen Verfahrens zeigt schematisch die *Fig. 3.* Die Hinweiszeichen bedeuten:

*B* Feldlinien des störenden magnetischen Flusses

*S₁, S₂* Vektoren $S_1 = (S_{x1}, S_{y1}, S_{z1})$ bzw. $S_2 = (S_{x2}, S_{y2}, S_{z2})$
Φ von den Meßsignalvektoren eingeschlossener Winkel nach der Formel **I.**

Unter der Annahme, dass Quellen des störenden magnetischen Flusses räumlich weiter entfernt sind, als der magnetische Körper bzw. die orale Darreichungsform, ist der Winkel zwischen den Vektoren $S_1$ und $S_2$ näherungsweise zeitlich konstant. Im besten Falle, nämlich in einem homogenen Magnetfeld, verschwindet dieser Winkel sogar konstant. Es wurde aber gefunden, dass störende Magnetfelder oft im Wesentlichen homogen sind. Ein Vorteil bei der Bestimmung des Winkels Φ ist die Unerheblichkeit fehlerhafter Ausrichtung einzelner oder aller AMR-Sensoren oder der Verkippung der Sensoranordnungen gegeneinander, wenn diese fehlerhafte Ausrichtung zeitlich konstant ist. Ein solcher Fehler macht sich in einem bedeutungslosen Offset im Φ/t - Diagramm bemerkbar, gleichbedeutend mit

$$\Phi = \text{const}$$

bezüglich der Zeit t.

In dem erfindungsgemäßen Verfahren kann im Schritt (b) zumindest ein Tiefpassfilter mit der Grenzfrequenz von 0,1 - 0,99 mHz, 1 mHz - 0,99 Hz, 1 Hz - 9,99 Hz, 10 Hz - 1 kHz, oder eine Kombination aus Tiefpassfiltern mit zumindest zwei verschiedenen Grenzfrequenzen eingesetzt werden. Dabei ist bevorzugt, die Filterung dem zu detektierenden Vorgang anzupassen, um Rauschen und/oder rasch veränderliche Störfelder, zum Beispiel durch Elektrogeräte, im Messsignal zu unterdrücken.

In dem erfindungsgemäßen Verfahren kann der Beitrag eines jeden AMR-Sensors oder das im Schritt (c) erhaltene Meßsignal durch einen Medianfilter gefiltert werden.

Des Weiteren kann in dem erfindungsgemäßen Verfahren während der Durchführung des Schrittes (c) die

erhaltene Größe $\Delta$ und/oder $\Phi$ als Funktion der Zeit durch einen Datalogger oder ein anderes geeignetes, dem Fachmann bekanntes Gerät aufgezeichnet werden, mit dem das erfindungsgemäße Detektorsystem ausgestattet sein kann. Diese Aufzeichnung kann kontinuierlich erfolgen, zum Beispiel während der Einnahme, der Passage und/oder der Zersetzung des magnetischen Körpers im Organismus des Trägers. Sie kann auch diskontinuierlich erfolgen, um beispielsweise Energie zu sparen.

Es wurde gefunden, dass viele alltägliche Quellen störender Felder charakteristische Linienformen in den $\Delta$/t - bzw. $\Phi$/t- Diagrammen erzeugen. So können zum Beispiel vorbeifahrende Kraftfahrzeuge, elektrische Schaltvorgänge, durch Funken verursachte elektromagnetische Störungen, sowie stochastisch periodische Störungen und/oder Bürstenfeuer von Elektromotoren im Diagramm erkannt und deren Beitrag zur Linienform durch dem Fachmann bekannte Software kompensiert werden.

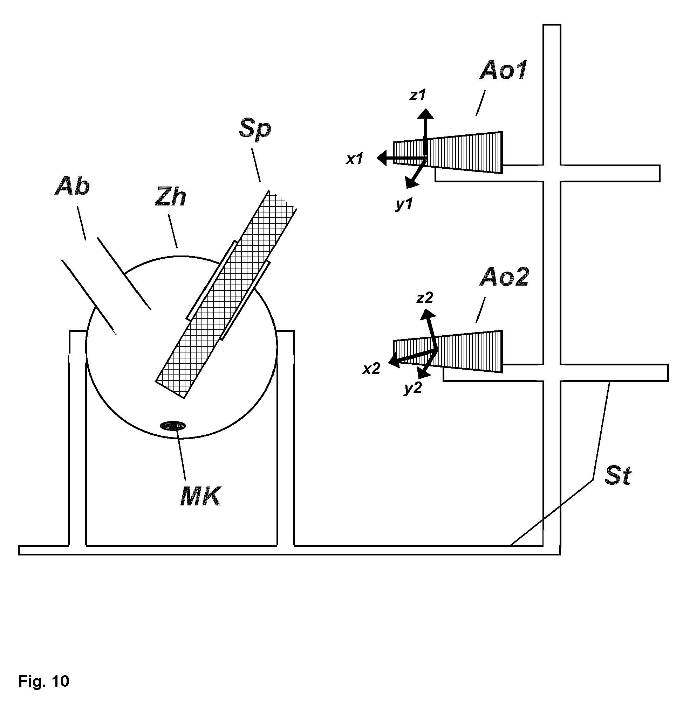

Das erfindungsgemäße Verfahren lässt sich vorteilhaft auch dann einsetzen, wenn sich der magnetische Körper bereits im Magen befindet und dort zerfällt. Der magnetische Körper kann auch im Darm oder im Colon zerfallen. In diesen Fällen wird eine digitale Filterung in dem Bereich 0,1 bis 1 mHz bevorzugt. Soll der Schluckvorgang erfasst werden, ist ein Tiefpassfilter mit einer Grenzfrequenzbereich von 1 mHz - 0,99 Hz bevorzugt. Des Weiteren kann es vorteilhaft sein, die Auswahl der Filter und/oder Grenzfrequenzen dem geometrischen Aufbau des magnetischen Körpers, insbesondere der oralen Darreichungsform anzupassen. Der Zeitraum, in welchem sich eine orale Darreichungsform, zum Beispiel eine Kapsel *(Fig.10)* zersetzt, liegt im Bereich von 0,5 - 30 min, bevorzugt im Bereich von 0,5 - 20 min, weiterhin bevorzugt im Bereich von 0,5 - 5 min. Sollen derart lang andauernde Vorgänge im menschlichen Körper gemessen werden, können die Signale bevorzugt "exponentiell geglättet" werden. Die mathematische Vorgehensweise hierfür ist dem Fachmann bekannt. Bevorzugte Glättungskonstanten $\alpha$ sind im Bereich von 0,10 bis 0,40, besonders bevorzugt liegt $\alpha$ bei ungefähr oder gleich 0,25.

Der magnetische Körper der Darreichungsform des erfindungsgemäßen Detektorsystems weist Untereinheiten auf, die Schichten, Phasen, und/oder Domänen sein können. Diejenige Untereinheit, die den magnetischen Fluss erzeugt, weist inerte, kristalline Teilchen auf, die Partikel, verglaste und/oder umhüllte Mikro- und/oder Minimagnete sein können. Vorzugsweise haben die Mikro- und/oder Minimagnete die Form von Zylindern, Schalen, und/oder Kugeln.

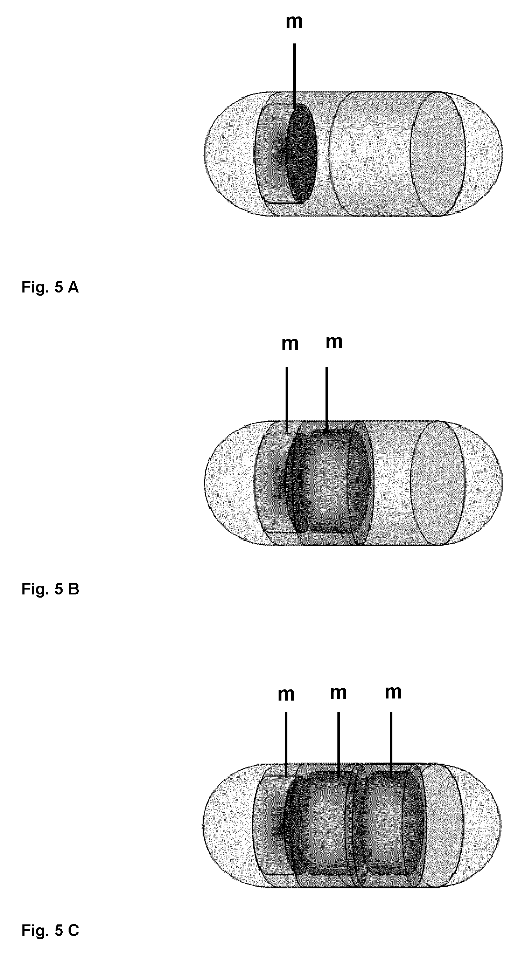

Bevorzugte Abmessungen der Mikro- bzw. Minimagnete sind von 0,1 bis 1 $\mu$m, von 1 bis 10 $\mu$m, von 10 bis 100 $\mu$m, von 100 $\mu$m bis 1 mm, und/oder von 1 mm bis 10 mm. Die Mikro- bzw. Minimagnete weisen magnetische Teilchen auf, vorzugsweise solche aus Magnetit und/oder einem magnetischen Material, welches der menschliche Organismus nicht verstoffwechselt. Des Weiteren können die magnetischen Teilchen mikrostrukturierte Polymerverbünde, und/oder teilkristalline, polymorphe, gesinterte, pulverförmige, oder Kombinationen davon aufweisen. Die magnetischen Teilchen können auch weitere, kommerziell gängige Komponenten aufweisen, vorzugsweise von diesen umhüllt sein, zum Beispiel von Dextranpartikeln, oder von anderen Komponenten für eine molekulare Umhüllung, zum Beispiel von Cyclodextrinen, oder von Komponenten, die durch Granulations- oder Pelletierverfahren erhalten sind. Indem die Mikro- bzw. Minimagnete durch diese verkapselt oder umhüllt sind, ist die systemische Aufnahme der Mikrobzw. Minimagnete inhärent behindert. Vorzugsweise wird durch diese die Zersetzung der Mikro- bzw. Minimagnete durch die Magensäure verlangsamt und/oder der Beginn der Zersetzung verzögert. Mit dem fortschreitenden Zerfall wiederum schwächt derjenige magnetische Fluss bis zum Verschwinden ab, der mit dem erfindungsgemäßen Detektorsystem gemäß dem erfindungsgemäßen Verfahren erfasst wird. Die *Figuren 5 A - C* zeigen bevorzugte Ausführungsformen des magnetischen Körpers, und zwar in Form einer Kapsel, die jeweils mit einem *(Fig. 5 A)*, zwei *(Fig. 5 B)*, oder drei *(Fig. 5 C)* Minimagneten (m) ausgestattet ist.

Der magnetische Körper wird vorzugsweise mittels dem Fachmann bekannter galenischer Verfahren für die Herstellung oraler Darreichungsformen hergestellt, zum Beispiel mittels GMP-fähiger Herstellungsverfahren, vorzugsweise für die Herstellung von *Granules* mittels sogenanntem *high shear mixer,* oder in einem Wirbelschichtgranulator, mittels *roller compactor,* einem Extruder, Spheronisator, oder einem hotmelt-Prozess. Weiterhin bevorzugt ist die Herstellung von sogenannten *Pellets* mittels dem Fachmann bekannnter *pelletization,* Extrusion und *spheronization, rotar granulation,* oder *powderlayering.* Weiterhin können magnetische Körper in Form von Mikrotabletten aus teilkristallinem, verpresstem, verkapseltem, und/oder tablettiertem Material hergestellt werden, indem diese aus Pulver und polymorphen Substanzen kompaktiert werden. Orale Darreichungsformen können weiterhin in Form dem Fachmann bekannter Briefchen, den sogenannten *Sachets,* hergestellt werden.

Auch sind komplexere Formen magnetischer Körper denkbar, in denen zum Beispiel der magnetische Anteil die Form eines oder mehrerer Filme aufweist. Magnetische Körper des erfindungsgemäßen Detektorsystems können in jeder beliebigen Kombination der oben genannten Verfahren erhalten werden. Diese können weiterhin Vielteilchen-Systeme, Mehrschichtsysteme, core-shell-Systeme, und/oder Co-Block-Systeme sein.

Die orale Darreichungsform kann jede beliebige Form aufweisen, die zumindest eine magnetische Phase aufweist, wobei *unter "magnetischer Phase"* ein im magnetischen Körper räumlich abgegrenzter Körper verstanden wird, der einen magnetischen Fluss verursacht. Dieser wird gemäß dem erfindungsgemäßen Verfahren erfasst. Die orale Darreichungsform wird nach Einnahme in den menschlichen Körper in einer definierten Zeitdauer zersetzt. Sind zum Beispiel zwei, drei, vier oder fünf magnetische Phasen enthalten, können diese Zeitdauern unterschiedlich lang, bevorzugt paarweise unterschiedlich lang sein. Die unterschiedliche Länge der Zeitdauern kann zum Beispiel dadurch erreicht werden, dass das magnetische Material in einen Polymerfilm gehüllt wird.

Ist die orale Darreichungsform eine Kapsel, kann beispielsweise eine Hälfte der Kapsel mit dem magnetischen Material befüllt sein. Weiterhin kann das magnetische Material zu einer Tablette gepresst in der Kapsel verbracht sein. Die magnetische Phase kann vorzugsweise von einer Hülle umgeben sein, die resistent gegen Magensäure ist und die mit der Hülle der oralen Darreichungsform zusammenfällt oder von dieser verschieden ist. Die Funktionen solcher langsam zerfallenden Hüllen, auch *"Überzüge" oder "Matrixstrukturen"* genannt, ist dem Fachmann bekannt. Mit dem Beginn des Zerfalls der Hüllen setzt selbstredend auch der Zerfall des magnetischen Materials dann ein, sobald dieses mit dem Medium in Kontakt kommt, das den Zerfall der Hülle bewirkt bzw. bewirkt hat. Mit dem Zerfall des magnetischen Materials geht die den magnetischen Fluss verursachende kollektive Ordnung der Elektronenspins verloren, und mit dem Verlöschen der kollektiven magnetischen Ordnung schwächt der magnetische Fluss bis zur Unmessbarkeit oder seinem Verschwinden ab.

Das Material einer langsam zerfallenden Hülle oder Verkapselung kann ausgewählt sein aus filmbildenden Polymeren. Diese können zum Beispiel Copolymere aus Methylmethacrylat und Ethylacrylat, Copolymere aus Methylmethacrylat und Ethylacrylat und Methacrylsäure, Copolymere aus Methylmethacrylat und Methylmethacylat und Methacrylsäure und Copolymere aus Methylmethacrylat, Ethylacrylat und Trimethylammoniumethylmethacrylat sein.

Insbesondere geeignet sind Copolymere vom Typ EUDRAGIT® E100, EUDRAGIT® E PO, EUDRAGIT® L100, EUDRAGIT® L100-55, EUDRAGIT® S, EUDRAGIT® FS, EUDRAGIT® RS oder EUDRAGIT® RL. EUDRAGIT® NE oder EUDRAGIT® NM.

Geeignet sind weiterhin Polyvinylpyrolidone (PVP), Polyvinylalkohole, Polyvinylalkohol-Polyethylenglycol-Graft-Copolymer (Kollicoat®), Stärke und deren Derivate, Polyvinylacetatphtalat (PVAP, Coateric®), Polyvinylacetat (PVAc, Kollicoat), Vinylacetat-Vinylpyrolidon-Copolymer (Kollidon® VA64), Vinylacetat : Crotonsäure-Copolymere, Polyethylenglykole mit einem Molekulargewicht über 1000 (g/mol), Chitosan, ein (Meth)acrylatcopolymer, bestehend aus 20 - 40 Gew.-% Methylmethacrylat und 60 bis 80 Gew.-% Methacrylsäure, bekannt als ist EUDRAGIT® S, eine vernetzte und/oder unvernetzte Polyacrylsäure, als Smartseal® bekannte Fissurenversiegeler auf Compositbasis, Salz der Alginsäure und/oder ein Pektin, Cellulosen wie z. B. anionische Carboxymethylcellulose und deren Salze (CMC, Na-CMC, Ca-CMC, Blanose, Tylopur), Carboxymethylethylcellulose (CMEC, Duodcell®), Hydroxyethylcellulose (HEC, Klucel), Hydroxypropylcellulose (HPC), Hydroxypropylmethylcellulose (HPMC, Pharmacoat, Methocel, Sepifilm, Viscontran, Opadry), Hydroxymehylethylcellulose (HEMC), Ethylcellulose (EC, Ethocel®, Aquacoat®, Surelease®), Methylcellulose (MC, Viscontran, Tylopur, Methocel), Celluloseester, Celluloseglycolat, Celluloseacetatphtalat (CAP, Cellulosi acetas PhEur, Celluloseacetate-Phtalate, NF, Aquateric®), Celluloseacetatsuccinat (CAS), Celluloseacetattrimeliat (CAT), Hydroxypropylmethylcellulosephtalat (HPMCP, HP50, HP55), Hydroxypropylmethylcelluloseacetatsuccinat (HPMCAS -LF, -MF, -HF) ist oder eine Mischung der genannten Polymere ist.

Zusätzlich zu den filmbildenden Polymeren können in sich bekannter Weise weitere pharmazeutisch übliche Hilfsstoffe, die keine filmbildenden Polymere sind, als Formulierungshilfsmittel eingesetzt werden oder zusätzlich enthalten sein. Hier sind beispielhaft Stabilisatoren, Farbstoffe, Antioxidantien, Netzmittel, Pigmente, Glanzmittel etc. zu nennen. Sie dienen vor allem als Verarbeitungshilfsmittel und sollen ein sicheres und reproduzierbares Herstellungsverfahren sowie gute Langzeitlagerstabilität gewährleisten. Weitere pharmazeutisch übliche Hilfsstoffe können in Mengen von 0,001 bis 30, bevorzugt 0,1 bis 10 Gew.-% bezogen auf das filmbildende Polymere vorliegen. Ebenfalls können dem Fachmann bekannte Hilfsstoffe für Tabletten, Kapseln, oder Arzneiformen eingesetzt werden.

Die orale Darreichungsform kann des Weiteren zumindest eine Schale und zumindest einen Kern aufweisen, welche die magnetischen Phasen sind und die im menschlichen Organismus der Reihe nach, von außen nach innen zersetzt werden, so dass der oder die Kerne den magnetischen Fluss am längsten aufrecht erhält oder erhalten.

Beispielsweise kann die Darreichungsform einen Kern in Form einer flachen Tablette aufweisen, wobei die flachen Seiten der Tablette die magnetische Phase sind, die mit einer weiteren Substanz fest verbunden, zum Beispiel chemisch oder mechanisch fixiert oder verschmolzen ist, und die dem menschlichen Organismus zugeführt werden soll. Diese Substanz kann zum Beispiel ein Wirkstoff, eine Arznei, oder allgemein eine biologisch aktive Substanz sein und an der Innenseite einer magnetischen Schale vorhanden sein. Die magnetischen Phasen der Tablette können verschieden dick oder in verschiedener Weise von einem weiteren Material ganz oder teilweise umhüllt sein, so dass die magnetischen Phasen binnen verschieden langer Zeitdauern zerfallen. Diese Zeitdauern können so gewählt sein, dass die magnetischen Phasen zerfallen, während die Darreichungsform im menschlichen Organismus transportiert wird, und somit jede magnetische Phase an einem anderen Ort im menschlichen Organismus zerfällt. Zum Beispiel kann eine Zeitdauer derart kurz gewählt sein, dass eine der magnetischen Phasen bereits während der Passage durch die Speiseröhre zerfällt.

In einer weiter bevorzugten Ausbildungsform kann die orale Darreichungsform zumindest drei Bestandteile aufweisen, von denen zumindest ein Bestandteil, vorzugsweise jedes Bestandteil eine magnetische Phase umschließt.

Die orale Darreichungsform kann des Weiteren zumindest drei Phasen aufweisen, von denen zumindest eine Phase eine biologisch aktive Substanz aufweisen kann, und die übrigen Phasen keine biologisch aktiven Substanzen enthalten, jedoch eine oder jeweils eine magnetische Phase. Solche Darreichungsformen lassen sich einfacher herstellen.

Die orale Darreichungsform kann ebenso bevorzugt eine magnetische Phase an oder auf ihrer äußeren Oberfläche aufweisen. Bei der Einnahme einer solchen Darreichungsform wird zunächst die magnetische Phase zersetzt. Danach erst kommen die übrigen Bestandteile der Darreichungsform mit dem menschlichen Organismus in Kontakt. Diese Ausführungsform hat nicht ausschließlich den Vorteil, dass das erfindungsgemäße Detektorsystem den genauen Zeitpunkt der Einnahme registriert. Der genaue Zeitpunkt der Einnahme kann zum Beispiel durch einen Peak in der zeitlichen Ableitung $\partial\Delta/\partial t$ der Meßsignalvektordifferenz erkannt werden, und/oder in einem plötzlichen Anstieg des Betrages von $\partial\Phi/\partial t$ über einen Wert hinaus, der zuvor festgelegt worden ist. Ein solcher Zeitpunkt ist im Rahmen der Erfindung gleichbedeutend mit der Detektion von sich verändernden Magnetfeldern und somit dem *Erkennen des Speiseröhren-Durchtritts.*

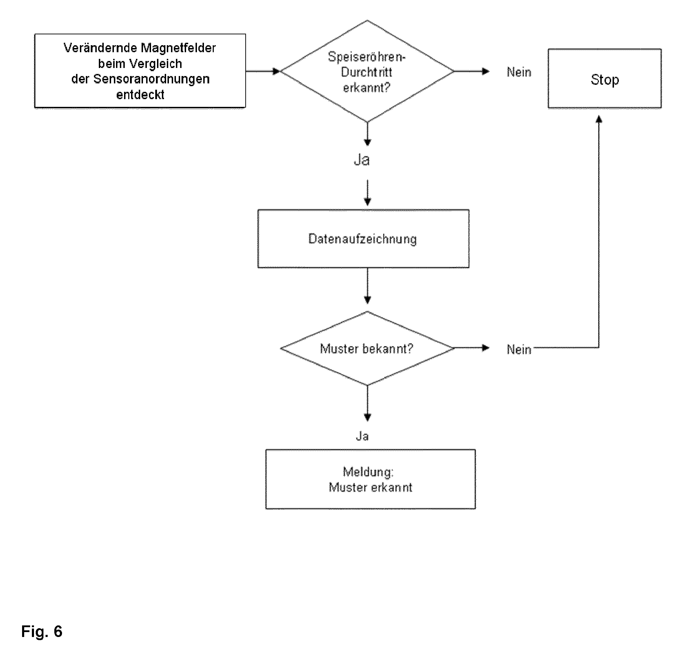

Falls das erfindungsgemäße Detektorsystem mit einem Gerät für die Aufzeichnung der Größe $\Delta$ und/oder $\Phi$ als Funktion der Zeit ausgestattet ist, ist das Erkennen der Speiseröhre, bezeichnet mit, *"Speiseröhren-Durchtritt erkannt"* logisch positiv. Die Prozessierung dieses und des folgenden logischen Zustandes ist in der ***Fig. 6*** schematisch dargestellt.

Wenn andererseits der Zeitpunkt der Einnahme bekannt ist, wurde als weiterer Vorteil dieser Darreichungsform gefunden, dass verschiedene externe magnetische Flüsse oder Flussänderungen, die zu verschiedenen Zeitpunkten vorliegen und im Schritt (b) und/oder (c) nicht vollständig ausgeblendet oder herausgerechnet werden können, dennoch als störende Flüsse erkannt werden, indem die Linienform, die der magnetische Fluss der Darreichungsform nach dem Zeitpunkt der Einnahme in dem $\Delta/t$ - oder $\Phi/t$ - Diagramm jeweils erzeugt, als jeweiliges Charakteristikum für das Diagramm herangezogen wird. Dies kann bewerkstelligt werden, indem unmittelbar nach der erstmaligen Einnahme der Darreichungsform die Linienform während eines Zeitintervalls von 0 bis 10 s, vorzugsweise von 0 bis 5 s, tabelliert und/oder durch geeignete mathematische Funktionen approximiert wird. Unmittelbar nach jeder weiteren Einnahme zu jeweils bekannten Zeitpunkten kann die dann erfasste Linienform mit der tabellierten bzw. approximierten Linienform verglichen werden. Ein solcher Vergleich wird im Rahmen der Erfindung mit *"Datenaufzeichnung und Datenabgleich"* bezeichnet. Stimmt die erfasste Linienform in ihrer tabellierten und/oder approximierten Form mit der Linienform bei der ersten Einnahme der Darreichungsform überein, so ist dieser Befund, bezeichnet *mit "Muster bekannt",* logisch positiv. Sind die logischen Werte *Speiseröhrendurchtritt erkannt* und *Muster bekannt* positiv, kann die Erfassung gemäß dem erfindungsgemäßen Verfahren vorgenommen werden, da nunmehr das vermittels der verändernden Magnetfelder gemessene *"Muster erkannt"* ist. Dann aber werden die weiteren Flussänderungen, die die Passage der Darreichungsform und deren Zersetzung im Organismus verursachen, trotz unterschiedlicher Umgebungen während verschiedener Zeitpunkte der Einnahme erfasst. Das ergibt den weiteren Vorteil einer Mobilität des erfindungsgemäßen Detektorsystems nahezu unabhängig von Ort oder Stärke externer magnetischer Flüsse, denn das erfindungsgemäße Verfahren unterscheidet nun sogar verschiedene, unbekannte externe Störeinflüsse. Ist wenigstens einer der beiden logischen Zustände negativ, kann die Erfassung vermieden, das erfindungsgemäße Detektorsystem ausgeschaltet, und/oder eine weitere Meldung generiert werden, die der Verwendung des Systems angepasst ist.

Gegenstand der Erfindung ist daher ebenfalls die Verwendung des erfindungsgemäßen Detektorsystems zur Erfassung geschluckter oraler Darreichungsformen und der Feststellung des oder der Zeitpunkte des Zerfalls des magnetischen, bevorzugt ferromagnetischen Anteils im Verdauungstrakt, wobei die orale Darreichungsform ein Lebens- oder Genussmittel ist und einen magnetischen Anteil aufweist. Der Vorteil besteht darin, dass zum Zeitpunkt des Zerfalls dieses Anteils, oder einen definierten Zeitraum davor, der magnetische Körper, allgemein die orale Darreichungsform ebenfalls zerfällt oder zerfallen sein muss, und somit darin eingeschlossene Substanzen freigesetzt sein müssen. Vorzugsweise kann bei der erfindungsgemäßen Verwendung der Zerfall des magnetischen, bevorzugt ferromagnetischen Anteils im Magen, Dickdarm, Dünndarm, und/oder Colon festgestellt werden. Eine Option der erfindungsgemäßen Verwendung ist wie folgt.

Weist der magnetische Körper zumindest zwei magnetische Phasen auf, deren Zeitpunkte des Zerfalls so gewählt sind, dass diese magnetischen Phasen an unterschiedlichen Orten im menschlichen Organismus zerfallen, und ist außerdem mit jeder dieser magnetischen Phasen je eine Substanz fest verbunden, die von dem menschlichen Organismus aufgenommen werden und zum Beispiel ein Wirkstoff, eine Arznei, oder allgemein eine biologisch aktive Substanz sein kann, und wird mit der Erfassung des jeweiligen Zerfalls außerdem eine Messung der Blutspiegel des oder der vom Organismus aufgenommenen Substanzen durchgeführt, dann kann zum Beispiel in klinischen Studien die Abgabe dieser Substanz oder Substanzen in vivo mit dem Verhalten des Metabolismus korreliert werden. Das erfindungsgemäße Detektorsystem kann demnach auch in einer Therapie und/oder Diagnostik verwendet werden, die entsprechenden therapeutischen oder diagnostischen Verfahren sind nicht Teil der Erfindung. Die vom Körper aufgenommene Substanz kann auch ein Lebens- und Genussmittel sein, und somit kann das erfindungsgemäße Detektorsystem in allen Bereichen der Ernährung verwendet werden.

Bei der erfindungsgemäßen Verwendung, können die gemäß des erfindungsgemäßen Verfahrens erhaltenen Meßsignale in einem Datenspeichergerät gespeichert, und die gespeicherten Daten vorzugsweise auf den Empfang eines Anforderungssignals hin an ein Empfangsgerät übermittelt werden.

Das Detektorsystem kann vorzugsweise die Signale über ein handelübliches Smartphone, Mobilphone, PDA übertragen, wobei durch einen weiteren Algorithmus an Bord dieses Kleincomputers eine Aufbereitung der Signale erfolgen kann. Ein Beispiel für eine solche Aufbereitung kann Datenreduktion, Verschlüsselung, und/oder Verrechnung mit persönlichen Daten des Trägers sein. Die vom erfindungsgemäßen Detektorsystem erhaltenen Signale können auf kabelgebundenem Wege, zum Beispiel vorübergehend mittels einer Steckverbindung, und/oder drahtlos übertragen werden, beispielsweise über Sensorknoten, Rechner, oder mittels Bluetooth®-Technologie auf ein mobiles Telefon. Nutzt man diese Technologie, kann der Aufwand für die Portierung der Software auf den digitalen Signalprozessor (DSP) eingespart und auch die Verarbeitungszeit verkürzt werden.

Das Datenspeichergerät kann ein Datalogger mit Sender sein, der zum Beispiel in Bluetooth®-Technologie ausgeführt sein kann. Ebenso ist denkbar, das erfindungsgemäße Detektorsystem mit einem Datalogger mit Sender, oder auch mit einem "Radio-Frequency Identification Device" (RFID) auszustatten. Mittels eines solchen Schaltkreises können bevorzugt einfach strukturierte Informationen gesendet und empfangen werden, beispielsweise solche gesendet werden, die mit einem besonderen Ereignis, z.B. einem Notfall verknüpft werden können. Diese Informationen können vorzugsweise aus den Meßsignalen abgeleitet werden, z.B. bei einem Missbrauch, Fehlverabreichung, zu häufiger oder zu seltener Dosierung, Unter- oder Überdosierung der oralen Darreichungsform, Energienotstand im oder Versagen des Detektorsystems. Auch können Systeme kombiniert werden, die bereits in der Medikation Anwendung finden, wie z.B. implantierte Schmerzmittelpumpen oder externe Perfusoren, die eine kontrollierte Injektion von Medikamenten steuern und wobei unter Umständen eine Kombination mit weiteren Medikamenten vermieden werden sollte.

Das Empfangsgerät kann jedes dem Fachmann bekannte Empfangsgerät sein, das von einem öffentlichen oder nicht öffentlichen Server, Rechner und/oder Netzwerk unterstützt wird. Die empfangenen Daten können über ein Netzwerk aus Mobilfunkgeräten, Rechnern, Workstations, Kleincomputern, oder jedem sonstigen Rechner oder Server verarbeitet werden, das diese Daten besonders bevorzugt zum Zwecke ärztlicher Betreuung aufbereitet und/oder speichert. Es kann weiterhin vorteilhaft sein, das erfindungsgemäße Detektorsystem in einem öffentlichen oder nicht-öffentlichen Datenmanagement Netzwerk zu verwenden, ebenso bevorzugt beim Datenmanagement oder in einem Datenmanagement Netzwerk im Rahmen einer Therapie und/oder Diagnostik.

Das Datenmanagement Netzwerk kann von Experten abgerufen oder benutzt werden. Wird beispielsweise ein Notfall signalisiert, kann über ein automatisiertes System, z.B. über ein *"computerized physician order entry system"* (CPOE), ein Experte angefordert werden, zum Beispiel ein Notarzt. Der Experte korreliert die vom Datenmanagement Netzwerk gesammelten Daten, um den Ort und Zeitpunkt des Ereignisses, z.B. des Notfalls zu ermitteln, sowie um geeignete Maßnahmen zu ergreifen.

Wird das erfindungsgemäße Detektorsystem in einer Therapie und/oder Diagnostik verwendet, kann das Datenmanagement Netzwerk vorteilhaft mit einem Pharmazie-Rechner oder einer pharmazeutischen Datenbank ausgestattet sein, ebenso vorteilhaft mit einem Expertensystem für Medikation.

Die vom erfindungsgemäßen Detektorsystem erhaltenen und gegebenenfalls gesendeten Signale können prozessiert, codiert und/oder gepackt in das Datenmanagement Netzwerk übertragen werden. Die in dieses Datennetz übertragenen Daten können auf kommerziellem Wege mittels Telefonanruf abgerufen werden. Die übermittelten Daten können mittelbar oder unmittelbar, in Echtzeit und/oder gespeichert den zeitaufgelösten Zerfall des magnetischen Körpers protokollieren, bestätigen, oder dem Fachmann bekannter Weise weitere Eingabeaufforderungen auslösen.

Datenmanagement Netzwerke für Therapie- und klinische Entwicklungen sind bekannt und erzeugen durch Verwendung von Expertensystemen, die beispielsweise neuronale Lernalgorithmen sind, höhere Datenqualitäten, und Kategorien als die Summe der Einzeldaten. Höheren Datenqualitäten aus großen statistischen Gesamtheiten können zum Beispiel auf der Basis von Datenreduktion oder Maximum-Entropie-Algorithmen gewonnen sein.

Bei der Verwendung des erfindungsgemäßen Detektorsystems und/oder dem Einsatz des erfindungsgemäßen Verfahrens in Netzwerksystemen können insbesondere kritische Patienten oder pflegebedürftige Personen vor Missbrauch, falscher Anwendung, oder sonstigen Gefahren im Zusammenhang mit der Applikation des magnetischen Körpers geschützt werden.

Das erfindungsgemäße Detektorsystem kann im Rahmen von Behandlungen, Untersuchungen, Diagnosen, sowie bei der Erforschung neuer Therapien und Diagnosen, und im Rahmen der Verknüpfung der medizintechnischen Systeme verwendet werden.

Ebenso kann das erfindungsgemäße Detektorsystem bei der Durchführung und Kontrolle von gastrointestinaler Wirkstoffdosierung, insbesondere in soliden oder solid-flüssig-Kombinationspräparaten, verwendet werden.

Des Weiteren kann es vorteilhaft sein, das Detektorsystem für Hochdurchsatz Tests zu verwenden. Mit Hilfe solcher Tests kann die Integrität der magnetischen Schichten, Phasen, und/oder Domänen geprüft, sowie das zeitliche Verhalten bei deren Auflösung im menschlichen Organismus bestimmt werden.

Die Erfindung wird im Folgenden anhand von Beispielen erläutert.

**Beispiele**

**Vergleichsbeispiel**

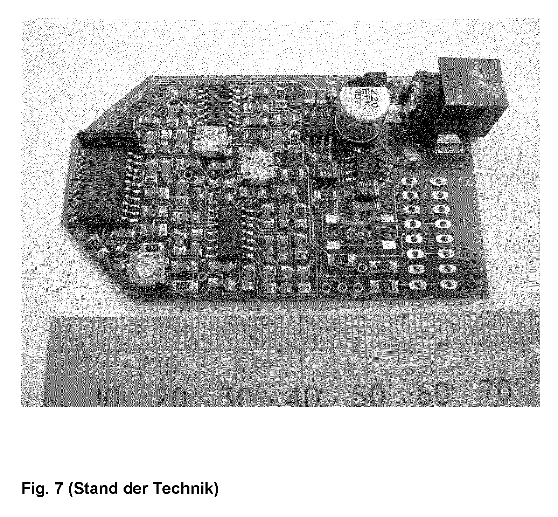

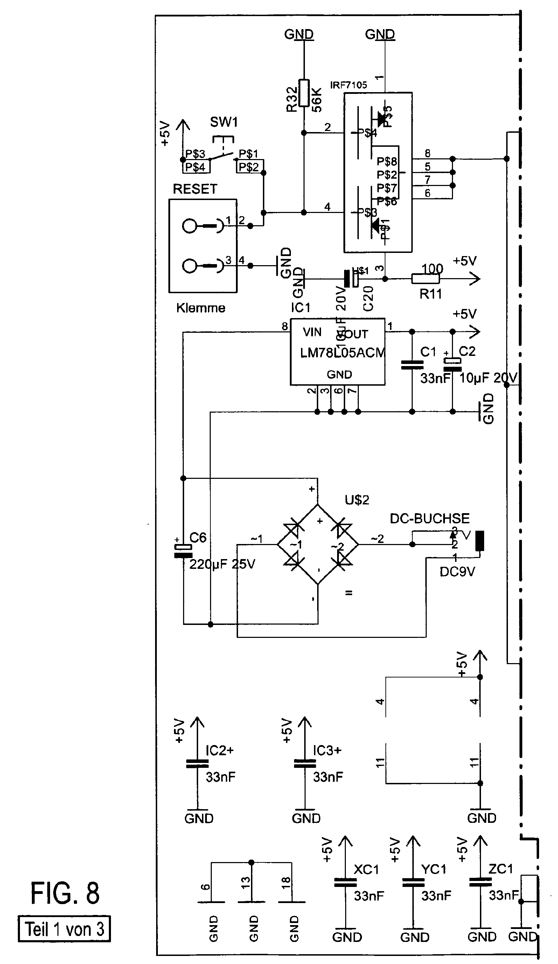

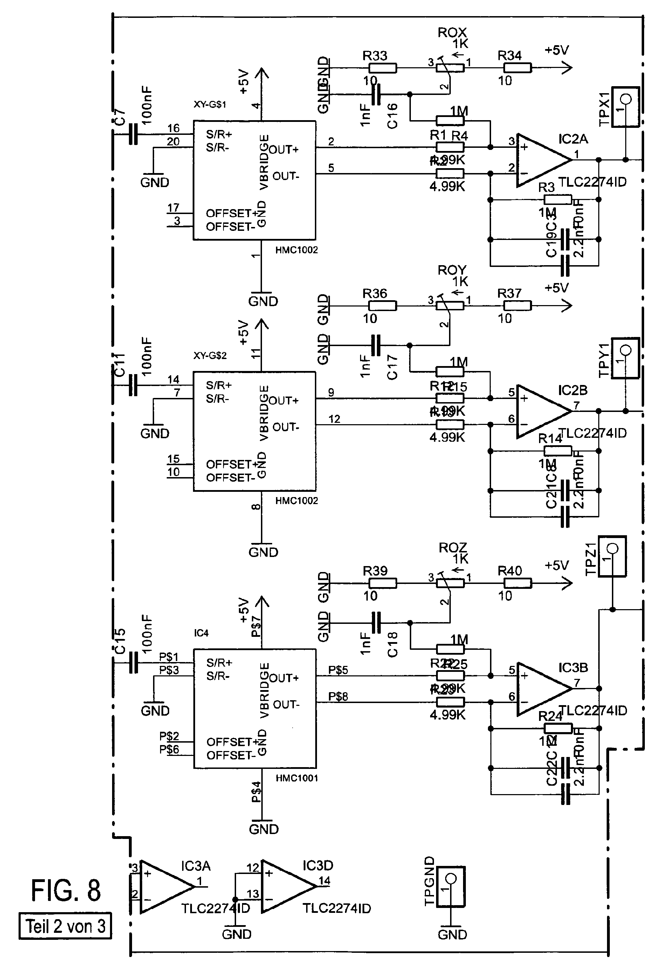

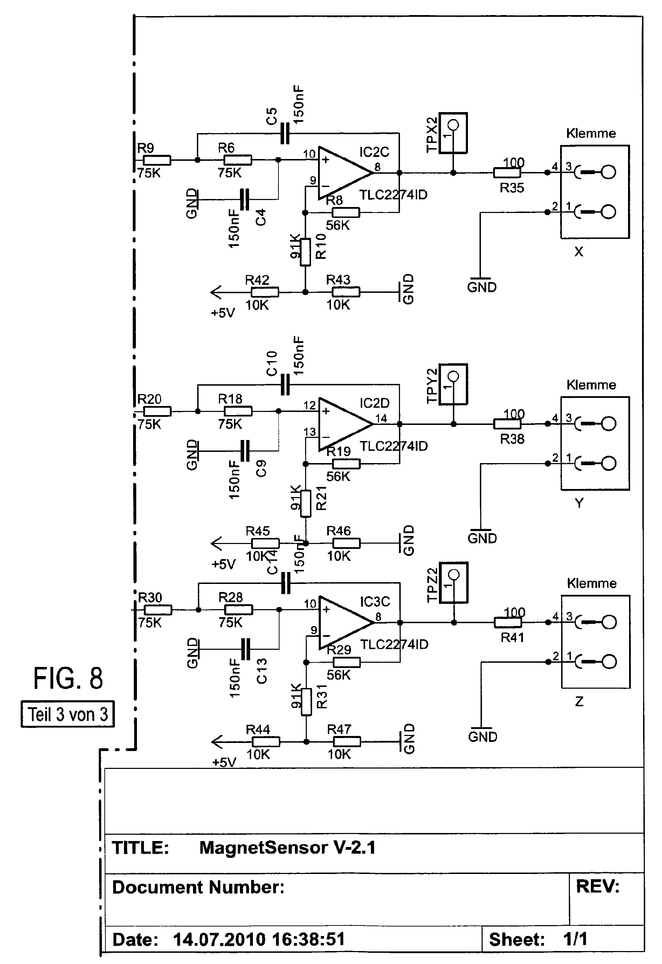

Es wurde eine Sensoranordnung mit drei AMR-Sensoren bereit gestellt. *Fig. 7* zeigt eine Ablichtung dieser Anordnung. Auf der linken Seite sind die AMR-Sensorschaltkreise zu sehen, deren Achsen der leichten Magnetisierung liegend parallel zur Leiterplattenebene bzw. senkrecht dazu standen. Der Schaltplan der Anordnung ist in *Fig. 8* dargestellt. Die verwendeten AMR-Sensoren verfügten zusätzlich zu den eigentlichen Sensorbrückenschaltungen noch über zwei interne Spulen pro Achse.

Die dargestellte Schaltung arbeitete ohne Offset-Strap und rein analog bei einer Versorgungsspannung von über 7 V. Digitalisierung und Signalverarbeitung erfolgten extern über einen Laptop mit angeschlossener USB-Multi-IO-Baugruppe des Typs NI USB-6211, und mittels einer Software von LabVIEW. Arbeitspunkt und Offset-Einstellung mussten manuell über Potentiometer erfolgen. Nur durch den Verzicht auf den Offset-Strap konnte eine geringe Stromaufnahme ermöglicht werden.

Diese Variante war für einen mobilen Einsatz am menschlichen Träger, der etliche Tage andauern muss, unbrauchbar oder zumindest umständlich, weil Arbeitspunkt und Offset-Einstellung immer wieder manuell vorgenommen werden mussten.

**Beispiel 1.**

**(a) Kapsel mit magnetischem Körper**

Es wurde zunächst der magnetische Körper hergestellt. Das Material:

- Hartgelatinekapseln, Größe "0"

- D-Fructose

- Magnetit

- EUDRAGIT® FS 30 D

- HCL 0,1 N

Die Minimagnete (*Fig. 5 A - C*, m) wurden als Magnetit enthaltende Tabletten, im Rahmen der Erfindung *"Magnetit-Tabletten"* genannt, ausgeführt. Sie wurden erhalten, indem zunächst Magnetit, Maisstärke, Magnesiumstearat und Kollidon auf dem Fachmann bekannte Weise miteinander vermischt wurden. Anschließend wurde das Gemisch in einer ebenfalls Stand der Technik gemäßen Weise zu Tabletten verpresst. Diese wurden mit einem Grundierungsüberzug versehen, sowie anschließend mit einem weiteren funktionellen Überzug ausgestattet, der den Zerfall der Magnetit-Tabletten bei Kontakt mit der Magensäure während einer definierten Zeitspanne verzögert.

Der funktionelle Überzug bestand aus einer Mischung aus SodiumLaurylsulfat, Stearinsäure, Talkum und Dibutylsebacat und EUDRAGIT® E PO in dem Fachmann bekannten Anteilen. Mithilfe eines handelsüblichen Trommelcoaters wurde die Dispersion über einen Zeitraum von wenigen Minuten bis zu einigen Stunden auf die vorher mit Hydroxypropylmethylcellulose (HPMC) als Grundierungsüberzug versehenen Magnetit-Tabletten aufgetragen. Je länger diese Zeitdauer war, desto dicker geriet das Coating. Die Menge dieses aufgetragenen Coatings ist in mg angegeben.

Die Dicke des erzielten Coatings war für die Zeitdauer maßgeblich, während der die beschichteten Magnetit-Tabletten ihrem Zerfall durch die die Magensäure widerstanden. Mithilfe unterschiedlicher Dicken des funktionellen Überzuges konnte somit die Verzögerung des Zerfalls der Magnetit-Tablette und somit das Verschwinden ihres magnetischen Flusses unterschiedlich gestaltet werden.

Eine halbe Hartgelatinekapsel wurde mit einer Spatelspitze Fructose befüllt. Auf eine Magnetit-Tablette wurden mit einer Feinpipette 5 μl EUDRAGIT® FS 30 D in die Mitte der Tablette als Tropfen aufgetragen. Mit Hilfe einer Pinzette wurde eine zweite Magnetit-Tablette deckungsgleich auf die Seite der Tablette mit dem EUDRAGIT® Tropfen gelegt und für etwa 10 Minuten trocknen gelassen, wobei die beiden Tabletten verklebten.

Die verklebten Tabletten wurden mithilfe einer Pinzette in die halbe Hartgelatinekapsel auf das Fructose-Pulver gesetzt. Diese Hälfte wurde mit Fructose aufgefüllt und das Pulver unter gelegentlichem Klopfen verdichtet. In die zweite Kapselhälfte wurde vorsichtig eine Spatelspitze Fructose eingefüllt. Anschließend wurden beide Kapselhälften ineinander gesteckt, so dass so wenig Fructose wie möglich heraus fiel.

Der innere Aufbau der so erhaltenen Kapsel wurde dokumentiert und in eine Magnetisiervorrichtung gesteckt.

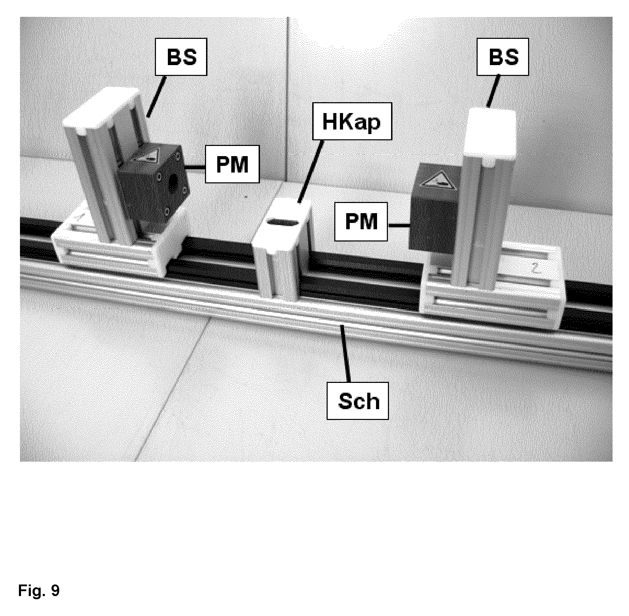

*Fig. 9* zeigt eine Ablichtung dieser Vorrichtung. Die mit den Magnetit-Tabletten versehene Hartgelatinekapsel wurde auf der Halterung (HKap) positioniert, so dass die Längsachse der Hartgelatinekapsel parallel zu der Schiene (Sch) lag. Anschließend wurden die beiden beweglichen Schlitten (BS), die mit Permanentmagneten (PM) ausgestattet und deren Magnetfelder parallel (Nord-Süd, Nord-Süd) zu der Schiene orientiert waren, an die Halterung mit der Kapsel herangeschoben. Dadurch befanden sich die Magnetit-Tabletten in dem resultierenden Magnetfeld und erhielten eine parallel dazu orientierte Magnetisierung. Es wurde gefunden, dass nach einer Verweilzeit von maximal 5 Minuten eine Sättigung der Magnetisierung erreicht wurde, die bei für magnetische Materialien und orale Darreichungsformen fachüblicher Lagerung permanent erhalten blieb. So erhielt man einen magnetischen Körper gemäß *Fig. 5B*.

Falls eine antiparallele Orientierung der Magnetfelder der Magnetit-Tabletten gewünscht wird, kann auch jede Magnetit-Tablette einzeln in der eben beschriebenen Vorrichtung magnetisiert werden. Anschließend können die Magnetit-Tabletten mit ihren Magnetpolen entgegen gesetzt verklebt und mithilfe einer Pinzette in die halbe Hartgelatinekapsel auf das Fructose-Pulver gesetzt werden, und anschließend der magnetische Körper zusammengesetzt werden. Bei dieser Orientierung der Magnetit-Tabletten sind jedoch Entmagnetisierungsphänomene zu befürchten.

**(b) Simulationsmodell**

Weiterhin wurde ein Simulationsmodell vorbereitet, das schematisch in *Fig.* **10** gezeigt ist. Ein Zweihalskolben (Zh), der den menschlichen Magen simuliert, wurde mit 300 ml 0,1 N Salzsäure befüllt, deren Temperatur auf 37 °C thermostatisiert war.

Der Zulauf zum Zweihalskolben simulierte die Speiseröhre (Sp). Über diesen Zulauf und den Ablauf (Ab) wurde ein Luftstrom geleitet, der die Flüssigkeit sowie den magnetischen Körper (MK) in sanfter Bewegung hielt, den man im Rahmen der Beispiele durch die Speiseröhre (Sp) in den Kolben (Zh) gleiten ließ. Diese Bewegung war die Simulation der menschlichen Bewegung und der Peristaltik des Magens.

Thermostatisierung, Befüllung, und Durchleitung eines Luftstromes sind in der *Fig. 10* nicht gezeigt. Von dem erfindungsgemäßen Detektorsystem zeigt die Abbildung lediglich die beiden Sensoranordnungen (Ao1) und (Ao2). Beide Sensoranordnungen waren dreikanalig ausgeführt, und *Fig. 10* zeigt die beiden Anordnungen zusammen mit jeweils den Achsen der leichten Magnetisierung, x1, y1, z1 bzw. x2, y2, z2. Über ein Stativ (St), das die Funktionen des Gurtes bzw. eines am Menschen mitgeführten Gerätes ersetzte, wurden Zweihalskolben und Sensoranordnungen zueinander in Position gehalten, sowie ein definierter Abstand von 10 cm und ein definierter Winkel von etwa 0 ° zwischen den beiden Sensoranordnungen erzielt. Dies simulierte das Tragen des erfindungsgemäßen Detektorsystems am Menschen.

Die Beträge der Vektoren $S_1$ und $S_2$ der erfindungsgemäßen Sensoranordnungen wurden ermittelt und voneinander subtrahiert. Bevor dies jedoch vorgenommen werden konnte, musste jede Sensoranordnung zunächst justiert und kalibriert werden.

**(c) AMR-Sensoren und deren Justage und Kalibrierung**

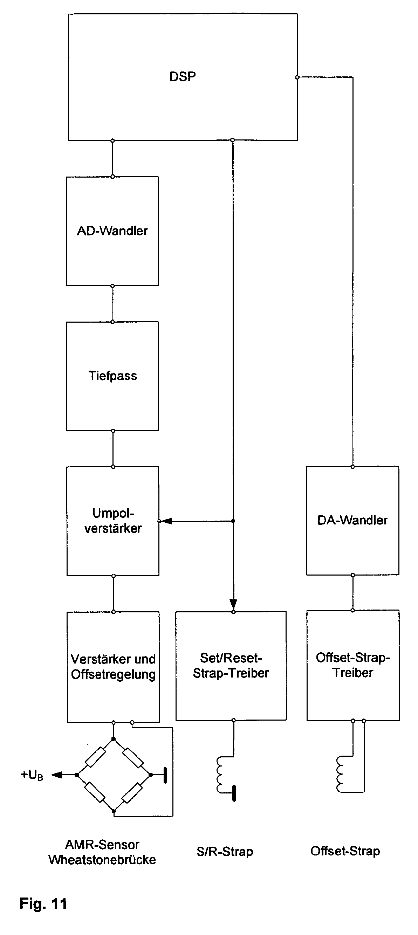

*Fig. 11* zeigt das Blockschaltbild für einen AMR-Sensor. Über einen Treiber wurden periodisch alternierend Set- und Reset-Impulse auf den Set-Reset-Strap des AMR-Sensors geschaltet, wodurch die Kennlinie des Sensors periodisch invertiert wurde. Durch diese Maßnahme wurde eine gegenüber einer Referenzspannung auftretende und vom magnetischen Fluss unabhängige Offsetspannung im nachfolgenden Verstärker automatisch ausgeregelt.

Über einen Umpolverstärker und einen Tiefpass wurde das eigentliche, durch die Set- bzw. Reset-Impulse modulierte Signal zurück gewonnen. Das Signal wurde anschließend digitalisiert und zur weiteren Verarbeitung dem DSP zugeführt. "DSP" ist die Abkürzung für einen digitalen Signalprozessor. Digitale Signalprozessoren sind im Gegensatz zu den Prozessoren für PC für die digitale Signalverarbeitung unter Echtzeitbedingungen optimiert. Sie sind dem Fachmann bekannt und werden z.B. für Sprach- und Bildverarbeitung und in der Messtechnik eingesetzt. Sie verfügen auf ihrem Chip beispielsweise über mehrere parallel einsetzbare Recheneinheiten, Speicher, diverse Zähler, Peripherie zur Kommunikation, universelle Schnittstellen, AD- und DA-Wandler, sowie eine andere Befehlsstruktur. In diesem Beispiel wurde also ein kompletter in einem Schaltkreis integrierter Rechner eingesetzt, nämlich eine ADSP-BF504F von *Analog Devices,* die eine Variante aus der Blackfin-Familie, *http://www.analog.com/en/processors-dsp/blackfin/adsp-bf504f/processors/product.html,* ist.

Mit Hilfe des Offset-Straps konnte das gemessene Magnetfeld teilweise kompensiert werden. Dazu wurde bei Über- oder Unterschreiten eines Schwellwerts durch Erhöhen oder Erniedrigen des Ausgabewertes für den DA-Wandler der Strom durch den Offset-Strap angepasst. Mit einem 8-Bit-DA-Wandler konnte so der Messbereich in 256 Segmente unterteilt werden. Dadurch konnte der Sensor im Bereich der größten Empfindlichkeit und Linearität bei gleichzeitig drastisch minimierter Querempfindlichkeit betrieben werden. Daneben verbesserte sich die Auflösung, da der Aussteuerbereich des AD-Wandlers auf die Größe eines Segmentes zuzüglich eines erforderlichen Überlappungsintervalls zwischen den Segmenten beschränkt wurde.

Da für die Kompensation magnetischer Felder über den Offset-Strap durch die geringe Windungszahl der integrierten Spulen relativ hohe Ströme erforderlich waren, hätte in dieser Betriebsart die mit einer vorgegebenen Akkumulatorkapazität erreichbare Betriebszeit sinken müssen. Abhilfe wurde jedoch dadurch geschaffen, dass die aktive Messzeit eines jeden AMR-Sensors verringert wurde und der Offset-Strap zwischen den Messungen immer über den DA-Wandler in den neutralen Bereich gefahren wurde.

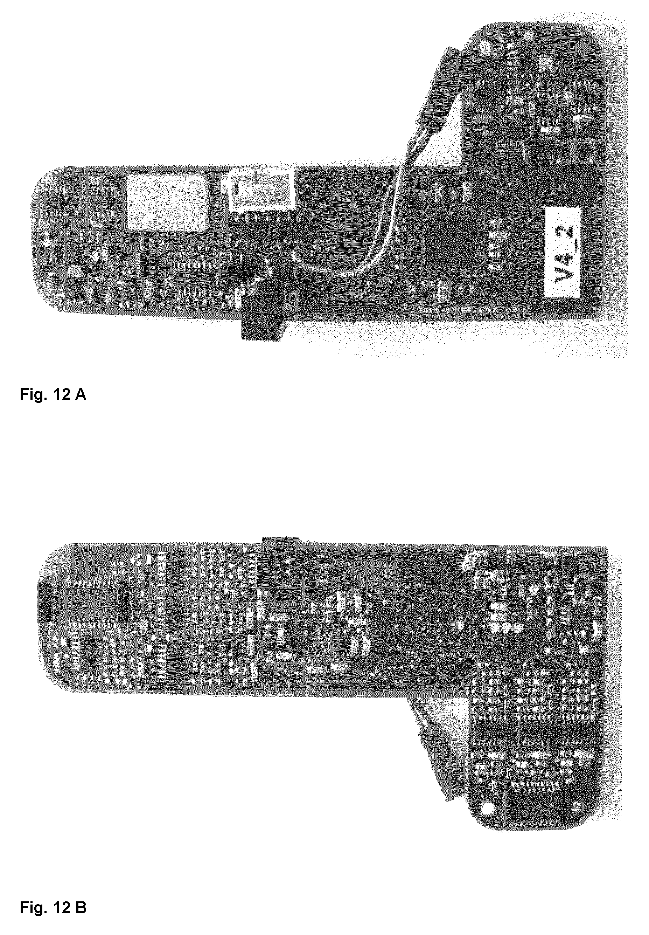

Die Oberseite einer der beiden gleich aufgebauten erfindungsgemäßen Sensoranordnungen des erfindungsgemäßen Detektorsystems zeigt die *Fig. 12 A,* und die Unterseite derselben die *Fig. 12 B.*

Im Allgemeinen streuen die Parameter der AMR-Sensoren und ihrer Bauelemente. Dadurch entstehen auch bei völlig gleich aufgebauter Elektronik Abweichungen der Messergebnisse, die nicht der unterschiedlichen Richtung oder Intensität von Magnetfeldern zugeordnet sind.

Solche Abweichungen führen schon bei einer einfachen Lageänderung der Sensoranordnung in einem homogenen Magnetfeld zu fehlerhaften Veränderungen der berechneten Beträge der Vektoren der magnetischen Flussdichten aus den einzelnen AMR-Sensoren erhaltenen Komponenten. Um eine Bewegung der Detektoranordnung im Magnetfeld auch während der Detektion schwacher Meßsignale zu ermöglichen, ohne dass dies zu systematischen Messfehlern führt, ist eine exakte Justage und Kalibrierung jedes AMR-Sensors notwendig. Im Idealfall müsste eine solche Justage in einem feldfreien Raum, einer sogenannten Nullkammer, erfolgen.

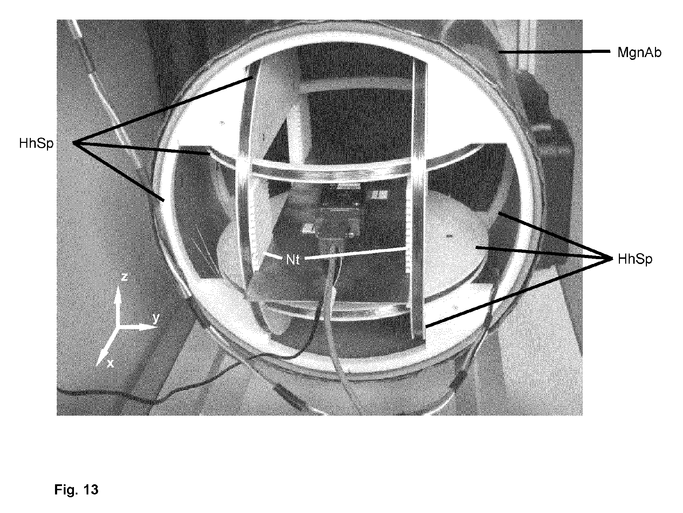

Um diese näherungsweise zu erzeugen, wurde für die AMR-Sensoren eine Anordnung aus drei Helmholtz-Spulenpaaren bereit gestellt, deren Spulenströme manuell eingestellt wurden. Diese Vorrichtung wurde in ein zylinderförmiges Gehäuse gebracht, das eine magnetische Abschirmung aus 5 Lagen 0,1 mm starker Mu-Metall-Folie aufwies. Das zylinderförmige Gehäuse war ein 600 mm langes KG-Rohr DN 300. Die *Fig. 13* zeigt diese Anordnung aus drei Helmholtz-Spulenpaaren (HhSp) und dem zylindrischen Teil der magnetischen Abschirmung (MgnAb), die an einem zylinderförmigen Gehäuse anlag. Die magnetische Flussdichte wurde auf dem Fachmann bekannte Weise mit einem Honeywell Magnetometer HMR 2300 gemessen.

Es wurde ein Schirmfaktor von mindestens 7,27 erreicht. Die Auswirkungen der konstruktiv bedingten Spalte an den Stirnseiten des zylinderförmigen Gehäuses auf den erreichbaren Schirmfaktor wurden durch eine 40 mm breite Überlappung zwischen dem Gehäuse und seiner Deckelabschirmung, die in *Fig. 13* nicht gezeigt ist, verringert. Die teilautomatisierte Durchführung der für die Justage erforderlichen Messungen ist dem Fachmann bekannt. Sie wurde mit Hilfe einer Ansteuerelektronik erreicht, die sowohl einen manuellen Nullpunktabgleich, als auch die Steuerung der Spulenströme durch DA-Wandler und die Messung der Ströme über AD-Wandler gestattete.

Im Zentrum der Spulenanordnung wurde ein homogenes Magnetfeld erzeugt, wobei der Bereich, in dem der Fehler keiner als 0,25 % war, von der Spulengröße abhing, und zwar in Achsenrichtung im z-Bereich von $\pm 2{,}6$ cm, und im x-Bereich von $\pm 3$ cm. Für einen Bereich von mindestens von $\pm 1{,}5$ cm in jeder Achsenrichtung wurden Abweichungen unter 0,03 % erreicht.

Der Abstand der Spulenpaare war unterschiedlich, da für die homogene Feldverteilung im Inneren eines jeden Paares Helmholtzspulen der Spulenabstand gleich dem Radius der Spulen sein muss. Da in der Spulenanordnung die Spulen ineinander verschachtelt sein mussten, mussten unterschiedliche Spulendurchmesser gewählt werden. Die Abstände der Spulen betrugen 135 mm, 126 mm bzw. 115 mm. Die Spulenkörper des Paares in y-Richtung (Dreibein in *Fig. 13)* verfügten über Nuten (Nt), in die Trägerplatinen mit den darauf fixierten Sensoranordnungen oder AMR-Sensoren eingeschoben werden konnten. Damit war eine sichere Positionierung der Trägerplatinen während der Justage gegeben.

Für die Messungen wurde eine Software erstellt, die berücksichtigte, dass für die verschiedenen Messobjekte, nämlich das Magnetometer mit Fluxgatesonde von Projektelektronik Berlin, das Magnetometer mit AMR-Sensoren von Honeywell, sowie die Sensorvarianten mit und ohne Segmentierung der Kennlinie, unterschiedliche Einstellungen sinnvoll waren. Die Software ermöglichte es, sowohl für eine frei wählbare Richtung x, y, oder z (Dreibein, *Fig. 13)* eine Stromrampe durchzufahren, als auch die sich aus diesem eingeprägten Strom erhaltenen Messwerte der im Zentrum der Spulenanordnung positionierten AMR-Sensoren aufzuzeichnen. Deren Achsen der leichten Magnetisierung wurden jeweils parallel zu einer der Richtungen x, y, oder z positioniert. Für die Stromrampe konnten obere und untere Grenze und die Schrittweite vorgegeben werden. Zusätzlich konnten die Wartezeiten nach bestimmten Ereignissen definiert werden. Diese Ereignisse waren das Erreichen des Startpunktes der Rampe, der Flip-Impuls an den AMR-Sensor, der Segmentwechsel des Sensors, Änderung des Spulenstromes. Die für die Justage ebenfalls erforderlichen Kennlinien der Spulen als Funktion jeweils des Spulenstromes wurden zuvor ermittelt, wobei eine Fluxgatesonde des Typs GeoX der Firma Projektelektronik, Berlin, und ebenfalls ein Magnetometer von Honeywell, Typ HMR 2300, eingesetzt wurden.

Der so erhaltene funktionale Zusammenhang zwischen den gemessenen AMR-Sensor Signalen und der mit dem Spulenstrom verknüpften magnetischen Induktion wurde auf dem Fachmann bekannte Weise für den gesamten Aussteuerbereich bzw. für alle 256 Segmente tabelliert. Aus den so erhaltenen Daten wurden Sensitivitäten und Querempfindlichkeiten berechnet und in Konfigurationsdateien übertragen.

Die Justierung der Sensoranordnungen ging allen folgenden Beispielen voraus. Die Aufnahme der Meßsignale der beiden Sensoranordnungen erfolgte mittels des grafischen Programmiersystems "LabView" (Firma National Instruments) auf einem handelsüblichen Computer. Die Meßdaten wurden automatisch in eine Excel Tabelle kopiert, von der aus die Daten graphisch dargestellt wurden.

**Beispiel 2.**

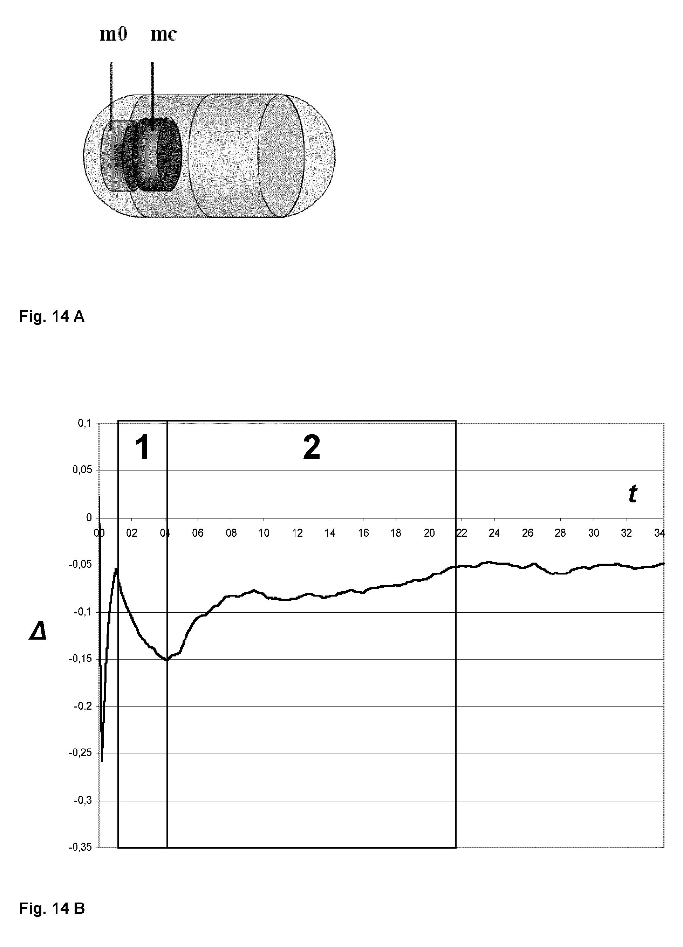

Es wurden zwei Magnetit-Tabletten hergestellt wie im Beispiel 1 (a), jedoch im Unterschied dazu eine dieser Tabletten mit einem Coating. Die fertige Kapsel ist schematisch dargestellt in *Fig. 14 A.* Eine der Magnetit-Tabletten (m0) erhielt kein Coating, so dass beim Kontakt mit der Salzsäure des Simulationsmodells keine Verzögerung der Auflösung eintrat. Die andere Magnetit-Tablette (mc) erhielt 2 mg Coating. Mit den beiden Magnetit-Tabletten wurde verfahren wie im Beispiel 1 (a), und anschließend wurde gemäß diesem Beispiel eine Hartgelatinekapsel zusammengesetzt.

Nach dem Anwenden der Magnetisiervorrichtung wurde die Kapsel in das Simulationsmodell gegeben.

Das Simulationsmodell (*Fig. 10*) wurde wie im Beispiel 1 (b) ausgeführt, und die Sensoranordnungen in einem Abstand von 10 cm und einem Winkel von 0° eingebaut. Die Justage der AMR-Sensoren wurde wie im Beispiel 1 (c) ausgeführt.

Die Meßsignale wurden ausgewertet, indem ein kleiner Kennlinienbereich um den Nulldurchgang mit hoher Auflösung genutzt wurde. Wurde ein vorgegebener Bereich über- oder unterschritten, gleichbedeutend mit einer drohenden Übersteuerung des AD-Wandlers, so wurde durch einen Strom durch den Offsetstrap der Bereich angepasst. Diese Anpassung erfolgte mit Hilfe eines 8-Bit Digital-Analog-Wandlers in 256 Stufen. Bei der Kalibrierung wurden für alle Sensorkanäle jeweils für alle 256 Segmente der Kennlinie Geradenapproximationen bestimmt und die entsprechenden Anstiege und Nulldurchgänge hinterlegt.

Die Meßsignale in x, y, z-Richtung der jeweiligen Sensoranordnung waren die Komponenten der Vektoren $S_1$ bzw. $S_2$ jeweils in Abhängigkeit von der Zeit. Die *Fig. 14 B* zeigt die Differenz $\Delta$ in $\mu$T der Beträge dieser Vektoren als Funktion der Zeit t in Minuten.

Zum Zeitpunkt t = 0 min wurde die Kapsel für einige Sekunden an die Speiseröhre (Sp) des Simulationsmodells gehalten, womit die orale Einnahme simuliert wurde. Durch die Speiseröhre glitt die Kapsel in den Kolben (Zh) des Simulationsmodells. Etwa zum Zeitpunkt t = 1 min in der Salzsäure Vorlage des Kolbens angelangt, zerfiel die erste Magnetit-Tablette während einer Zeitdauer von etwa 3 min vollständig, was daran zu erkennen war, dass $\Delta$ ein lokales Minimum erreichte. Das Verhalten der Differenz $\Delta$ war im Intervall (1) durch das mit ihrem Zerfall einhergehende allmähliche Abschwächen des magnetischen Flusses der ersten Magnetit-Tablette ohne Coating gegenüber der zweiten, noch völlig intakten Tablette bestimmt. Ab diesem Minimum zum Zeitpunkt etwa t = 4 min detektierte das erfindungsgemäße System demzufolge nur noch den magnetischen Fluss der zweiten Magnetit-Tablette, die mit 2 mg Coating beschichtet war, und die während weiterer 18 min zerfiel, zu erkennen in der Annäherung von $\Delta$ an den in etwa zeitlich konstanten Verlauf ab Ende des Zeitintervalls (2).

**Beispiel 3.**

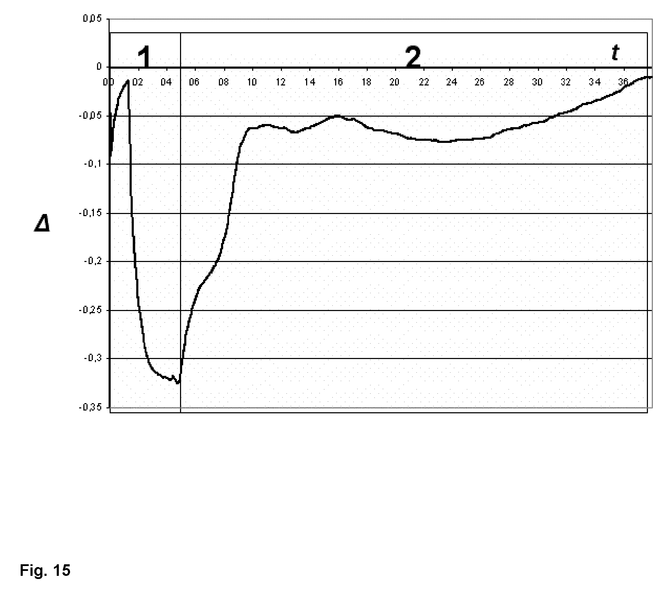

Dieses Beispiel wurde wie Beispiel 2 durchgeführt, jedoch im Unterschied dazu mit Magnetit-Tabletten, die mit anderen Coatings versehen waren. Eine Magnetit-Tablette erhielt 2 mg Coating, die andere dagegen 4 mg Coating. Das Verhalten der Differenz $\Delta$ als Funktion der Zeit ist in *Fig. 15* dargestellt.

Die orale Einnahme, die Beschickung des Simulationsmodells, und der Zerfall der mit 2 mg Coating versehenen Magnetit-Tablette erbrachte den zeitlichen Verlauf im Intervall (1). Man beobachtete den Zerfall der ersten Magnetit-Tablette bis etwa zu der Zeit t = 5 min. Ab Ende des Intervalls (1) setzte der Zerfall der mit 4 mg Coating versehenen zweiten Magnetit-Tablette ein, der etwa zum Zeitpunkt t = 37 min zum Ende des Intervalls (2) abgeschlossen war.

**Beispiel 4.**

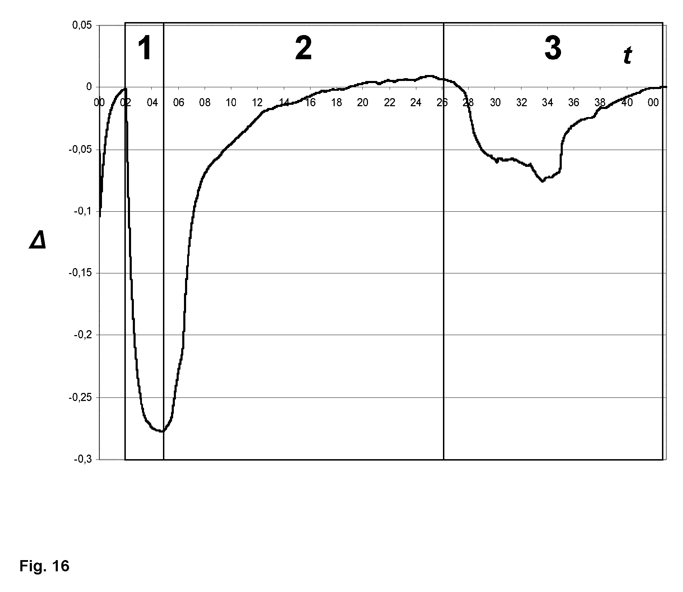

Dieses Beispiel wurde wie Beispiel 2 durchgeführt, jedoch im Unterschied dazu mit drei mit unterschiedlichem Coating versehenen Magnetit-Tabletten. Die Anordnung dieser Magnetit-Tabletten in der Kapsel war wie in *Fig. 5 C* gezeigt. Eine Magnetit-Tablette erhielt kein Coating, eine zweite 2 mg Coating, die dritte dagegen 6 mg Coating. Das Verhalten der Differenz $\Delta$ als Funktion der Zeit ist in *Fig. 16* dargestellt.

Die orale Einnahme und die Beschickung des Simulationsmodells waren zum Zeitpunkt t = 2 min abgeschlossen. Man beobachtete den Zerfall der nicht mit Coating versehenen Magnetit-Tablette bis zum Zeitpunkt t = 5 min während des Intervalls (1). Somit überwogen die magnetischen Flüsse der beiden verbliebenen Magnetit-Tabletten, von denen die mit 2 mg Coating versehene bis zum Ende des Intervalls (2) während folgender etwa 21 min zerfiel. Die dritte Magnetit-Tablette mit 6 mg Coating war während weiterer 16 min im Laufe des Intervalls (3) zerfallen.

**Beispiel 5.**

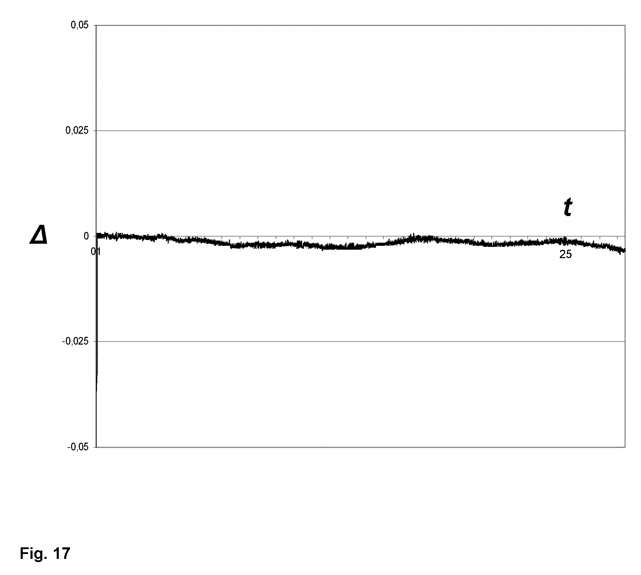

Dieses Beispiel wurde durchgeführt, wie Beispiel 2, jedoch ohne Magnetit-Tabletten. Die Differenz Δ als Funktion der Zeit während 25 min zeigte geringe Schwankungen um einen magnetischen Fluss nahe Null. Es wird vermutet, dass die geringen Werte aufgrund einstreuender Reste bzw. Spuren des umgebenden störenden Magnetfeldes zu messen waren. Das Resultat, dargestellt in *Fig.* **17,** plausibilisiert die Abwesenheit des magnetischen Körpers.

**Beispiel 6.**

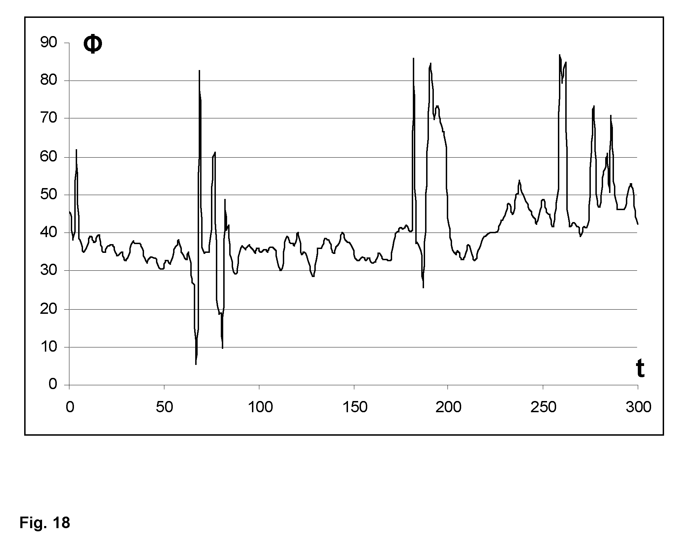

Dieses Beispiel wurde durchgeführt wie Beispiel 2, jedoch waren die Sensoranordnungen in einem Winkel von 45 ° gegeneinander verkippt, die Hartgelatinekapsel als der magnetische Körper wies lediglich eine Magnetit-Tablette auf, und der Kolben (Zh) des Simulationsmodells war ohne Salzsäure Vorlage, so dass ein Zerfall nicht möglich war. Die *Fig.* **18** zeigt den Winkel $\Phi$, $\Phi$ = arccos($S1 \cdot S2$ / $|S_1\|S_2|$), aufgetragen in Gradeinheiten als Funktion der Zeit in Sekunden.

Zum Zeitpunkt t = 0 s wurde die Kapsel für einige Sekunden an die Speiseröhre (Sp) des Simulationsmodells gehalten, womit die orale Einnahme simuliert wurde. Etwa bei der Zeit t = 5 s glitt die Kapsel an den Sensoranordnungen vorbei, so dass sich der Winkel $\Phi$ kurzzeitig auf etwa 60 ° veränderte. Zwischen den Zeitpunkten bei etwa t = 60 bis 90 s, 170 s bis 210 s, und ab etwa 260 s wurde der in den Kolben (Zh) eingeleitete Luftstrom derart erhöht, dass die Bewegung der Hartgelatinekapsel von gelegentlichem Rollen und Kippen in deutlich stärkere Dreh- und Taumelbewegungen überging.

**Patentansprüche**

**1.** Detektorsystem zur Erfassung magnetischer Körper im menschlichen Organismus, aufweisend

zumindest zwei Sensoranordnungen, wobei jede Sensoranordnung
einen, zwei, oder drei anisotrope Magnetwiderstandssensoren aufweist, deren Achsen der leichten Magnetisierung in paarweise unterschiedliche Richtungen weisen,
und jede Sensoranordnung von der oder den übrigen Sensoranordnungen einen Abstand von 0,5 bis 50 cm aufweist,
und
zumindest zwei Sensoranordnungen in einem Winkel von 0 bis 45 ° gegeneinander verkippt sind, **dadurch gekennzeichnet, dass**. die Sensoranordnungen in einem kombinierten Brust- und Schultergurt integriert sind.

**2.** Detektorsystem nach Anspruch 1, wobei

zumindest einer, bevorzugt jeder anisotrope Magnetwiderstandssensor 4 Barberpole-Elemente,
die zu einer Wheatstonebrücke oder einer Wheatstonebrückenersatzschaltung zusammen geschaltet sind,
und einen Set-Reset-Strap und einen Offset-Strap aufweist.

**3.** Detektorsystem nach einem der vorhergehenden Ansprüche, wobei der magnetische Körper eine Kapsel oder Kapsel mit Funktion ist, wobei
die Funktion ausgewählt ist aus Diagnostikum und/oder Arzneiform, und
die durch den Menschen geschluckt wird, und
die zumindest einen ferromagnetischen Anteil aufweist, vorzugsweise
zumindest einen Magnetit enthaltenden Kern und/oder Mantel.

**4.** Verfahren zur Erfassung der von einem magnetischen Körper im menschlichen Organismus erzeugten magnetischen Flussdichte durch ein Detektorsystem nach einem der Ansprüche 1 - 2, zur Erfassung oraler Darreichungsformen, wobei die orale Darreichungsform ein Lebens- oder Genussmittel ist und einen magnetischen Anteil aufweist, und zur Feststellung des oder der Zeitpunkte des Zerfalls des magnetischen Anteils im Verdauungstrakt, **gekennzeichnet durch** die Schritte

(a) zumindest einmaliges Aufschalten eines Set- und Reset-Impulses auf jeden anisotropen Magnetwiderstandssensor,
(b) Verstärkung der Signale jedes AMR-Sensors über eine geeignete Signalaufbereitung und über zumindest einen Tiefpassfilter,

(c) Bestimmung die Differenz der Beträge der Vektoren der magnetischen Flussdichten jeder Sensoranordnung,

und/oder
Bestimmung des Winkels Φ zwischen den Vektoren aus den Meßsignalen der AMR-Sensoren.

5. Verfahren nach Anspruch 4, wobei
der Beitrag eines jeden AMR-Sensors oder das im Schritt (c) erhaltene Meßsignal durch einen Medianfilter gefiltert wird.

6. Verfahren nach Anspruch 4, wobei
im Schritt (b) zumindest ein Tiefpassfilter mit der Grenzfrequenz von 0,1 - 0,99 mHz, 1 mHz - 0,99 Hz, 1 Hz - 9,99 Hz, 10 Hz - 1 kHz, oder eine Kombination aus Tiefpassfiltern mit zumindest zwei verschiedenen Grenzfrequenzen eingesetzt wird.

7. Verwendung des Detektorsystems nach einem der Ansprüche 1 - 2 in der Ernährung zur Erfassung oraler Darreichungsformen und der Feststellung des oder der Zeitpunkte des Zerfalls des magnetischen Anteils im Verdauungstrakt, wobei die orale Darreichungsform ein Lebens- oder Genussmittel ist und einen magnetischen Anteil aufweist.

8. Verwendung nach Anspruch 7, wobei die gemäß des Verfahrens nach einem der Ansprüche 4 bis 6 erhaltenen Meßsignale in einem Datenspeichergerät gespeichert, und die gespeicherten Daten vorzugsweise auf den Empfang eines Anforderungssignals hin an ein Empfangsgerät übermittelt werden.

9. Verwendung nach Anspruch 7 oder 8 in einem Datenmanagement Netzwerk.

## Claims

1. Detector system for detecting magnetic bodies in the human organism, comprising
   at least two sensor assemblies, wherein each sensor assembly
   has one, two or three anisotropic magnetic resistance sensors, of which the
   axes of weak magnetization point in different directions in pairs,
   and each sensor assembly has a spacing
   of 0.5 to 50 cm
   from the sensor assembly or the other sensor assemblies,
   and
   at least two sensor assemblies are tilted at an angle of 0 to 45° with respect to one another, **characterized in that** the sensor assemblies are integrated in a combined breast and shoulder strap.

2. Detector system according to Claim 1, wherein
   at least one, preferably each anisotropic magnetic resistance sensor has
   4 barber pole elements,
   which are connected together to form a Wheatstone bridge or a Wheatstone bridge equivalent circuit, and a set-reset strap and an offset strap.

3. Detector system according to one of the preceding claims, wherein the magnetic body is a capsule or capsule with function, wherein
   the function is chosen from diagnostic and/or pharmacological form, and
   which is swallowed by the person, and
   which has at least one ferromagnetic component, preferably
   at least one core and/or shell containing magnetite.

4. Method for detecting the magnetic flux density produced by a magnetic body in the human organism by means of a detector system according to one of Claims 1 - 2, for detecting oral administration forms, wherein the oral administration form is a food or stimulant and has a magnetic component, and for determining the time or times of the disintegration of the magnetic component in the digestive tract,
   **characterized by** the steps

   (a) at least once connecting a set and reset pulse to each anisotropic magnetic resistance sensor,

(b) amplifying the signals from each AMR sensor via suitable signal conditioning and via at least one low pass filter,

(c) determining the difference between the magnitudes of the vectors of the magnetic flux densities from each sensor assembly,

and/or

determining the angle Φ between the vectors from the measured signals from the AMR sensors.

5. Method according to Claim 4, wherein

the contribution of each AMR sensor or the measured signal obtained in step (c) is filtered by a median filter.

6. Method according to Claim 4, wherein

in step (b) at least one low pass filter having the cut-off frequency of 0.1 - 0.99 mHz, 1 mHz - 0.99 Hz, 1 Hz - 9.99 Hz, 10 Hz - 1 kHz, or a combination of low pass filters having at least two different cut-off frequencies is used.

7. Use of the detector system according to one of Claims 1 - 2 in nutrition for detecting oral administration forms and for determining the time or times of the disintegration of the magnetic component in the digestive tract, wherein the oral administration form is a food or stimulant and has a magnetic component.

8. Use according to Claim 7, wherein the measured signals obtained in accordance with the method according to one of claims 4 to 6 are stored in a data storage device, and the stored data is preferably transmitted to a receiving device upon the receipt of a request signal.

9. Use according to Claim 7 or 8 in a data management network.

**Revendications**

1. Système de détecteur destiné à détecter des objets métalliques dans l'organisme humain, présentant au moins deux agencements de capteur, dans lequel chaque agencement de capteur présente un, deux ou trois capteurs magnétorésistifs anisotropes, dont les axes de la légère magnétisation pointent dans des directions différentes par paires, et chaque agencement de capteur présente par rapport à l'autre ou aux autres agencement(s) de capteur une distance de 0,5 à 50 cm, et au moins deux agencements de capteur sont inclinés l'un par rapport à l'autre d'un angle de 0 à 45°, **caractérisé en ce que** les agencements de capteur sont intégrés dans une ceinture pectorale et scapulaire combinée.

2. Système de détecteur selon la revendication 1, dans lequel au moins un, de préférence chaque capteur magnétorésistif présente 4 éléments en poteau de barbier, qui sont connectés les uns aux autres en un pont de Wheatstone ou un circuit de remplacement de pont de Wheatstone, et une sangle de réglage-remise à zéro et une sangle déportée.

3. Système de détecteur selon l'une quelconque des revendications précédentes, dans lequel l'objet magnétique est une capsule ou une capsule avec une fonction, dans lequel la fonction est sélectionnée parmi un diagnostic ou une forme de médicament, et est avalée par la personne, et qui présente au moins une partie ferromagnétique, de préférence au moins un noyau et/ou une enveloppe contenant de la magnétite.

4. Procédé de détection de la densité de flux magnétique produite dans l'organisme humain par un objet magnétique au moyen d'un système de détecteur selon l'une des revendications 1 à 2, pour la détection de formes d'administration orales, dans lequel la forme d'administration orale est un aliment ou un stimulant et présente une partie magnétique, et pour la constatation du ou des instant(s) de la décomposition de la partie magnétique dans le tractus digestif, **caractérisé par** les étapes suivantes :

(a) appliquer au moins une fois une impulsion de réglage et remise à zéro à chaque capteur magnétorésistif anisotrope,
(b) amplifier les signaux de chaque capteur MRA par une préparation appropriée des signaux et au moyen d'au moins un filtre passe-bas,
(c) déterminer la différence entre les valeurs des vecteurs des densités de flux magnétique de chaque agencement de capteur, et/ou

déterminer l'angle φ entre les vecteurs à partir des signaux de mesure des capteurs MRA.

5. Procédé selon la revendication 4, dans lequel la contribution de chaque capteur MRA ou le signal de mesure obtenu à l'étape (c) est filtré(e) par un filtre médian.

6. Procédé selon la revendication 4, dans lequel on utilise à l'étape (b) au moins un filtre passe-bas avec la fréquence limite de 0,1 - 0,99 mHz, 1 mHz - 0,99 Hz, 1 Hz - 9,99 Hz, 10 Hz - 1 kHz, ou une combinaison de filtres passe-bas présentant au moins deux fréquences limites différentes.

7. Utilisation du système de détecteur selon l'une des revendications 1 à 2 dans l'alimentation pour la détection de formes d'administration et pour la constatation du ou des instant(s) de la décomposition de la partie magnétique dans le tractus digestif, dans laquelle la forme d'administration orale est un aliment ou un stimulant et présente une partie magnétique.

8. Utilisation selon la revendication 7, dans laquelle les signaux de mesure obtenus suivant le procédé selon l'une quelconque des revendications 4 à 6 sont stockés dans une mémoire de données, et les données stockées sont transmises à un appareil récepteur de préférence à la réception d'un signal de demande.

9. Utilisation selon la revendication 7 ou 8 dans un réseau de gestion de données.

# f=192Hz

FIG. 1    Teil 1 von 2

## Flipsteuerung

| TITLE: | MagnetSensor V-4.0_Pat | |
|--------|------------------------|-----|
| Document Number: | | REV: |
| Date: 23.11.2011 10:28:44 | | Sheet: 3/9 |

FIG. 1  Teil 2 von 2

FIG. 2   Teil 1 von 2

23

$$Imax = 5.0V / (10 + 2.5) = 0.4A$$

$$Uin = 2.5V \to I = 0.1A \to V = 0.25$$

$$RSX1/RSX2 = 0.25$$

## Offset Strap Treiber

| | | |
|---|---|---|
| TITLE: | MagnetSensor V-3.0 | |
| Document Number: | | REV: |
| Date: 17.11.2010 08:25:41 | Sheet: | 5/5 |

FIG. 2    Teil 2 von 2

Fig. 3

Gurt

Sensoranordnung einkanalig

Sensoranordnung dreikanalig

Fig. 4

m

Fig. 5 A

m    m

Fig. 5 B

m   m   m

Fig. 5 C

```
┌─────────────────────────┐                                                    ┌──────────────┐
│  Verändernde Magnetfelder│           ◇ Speiseröhren- ◇                        │              │
│      beim Vergleich      │─────────▶ ◇   Durchtritt   ◇──── Nein ──────────▶ │     Stop     │
│  der Sensoranordnungen   │           ◇   erkannt?     ◇                       │              │
│        entdeckt          │           ◇               ◇                        └──────────────┘
└─────────────────────────┘                  │                                         ▲
                                             │                                         │
                                             Ja                                        │
                                             │                                         │
                                             ▼                                         │
                                  ┌─────────────────────┐                              │
                                  │   Datenaufzeichnung  │                              │
                                  └─────────────────────┘                              │
                                             │                                         │
                                             ▼                                         │
                                      ◇ Muster bekannt? ◇──── Nein ───────────────────┘
                                             │
                                             Ja
                                             │
                                             ▼
                                  ┌─────────────────────┐
                                  │      Meldung:        │
                                  │   Muster erkannt     │
                                  └─────────────────────┘
```

Fig. 6

28

Fig. 7 (Stand der Technik)

FIG. 8

Teil 1 von 3

FIG. 8

Teil 2 von 3

FIG. 8

Teil 3 von 3

| TITLE: | MagnetSensor V-2.1 | | |
|---|---|---|---|
| Document Number: | | | REV: |
| Date: 14.07.2010 16:38:51 | | Sheet: 1/1 | |

Fig. 9

Fig. 10

**Fig. 11**

Fig. 12 A

Fig. 12 B

Fig. 13

**Fig. 14 A**

**Fig. 14 B**

Fig. 15

**Fig. 16**

Fig. 17

Fig. 18

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

• WO 2011026808 A1

**In der Beschreibung aufgeführte Nicht-Patentliteratur**

• Every slow-wave impulse is associated with motor activity of the human stomach. American Physiological Society, 08. Dezember 2008

• **CHAO HU.** A Cubic 3-Axis Magnetic Sensor Array for Wirelessly Tracking Magnet Position and Orientation. *IEEE Sensors Journal,* Mai 2010, vol. 10 (5